(19) Europäisches Patentamt / European Patent Office / Office européen des brevets

(11) **EP 2 645 098 B1**

(12) **EUROPEAN PATENT SPECIFICATION**

(45) Date of publication and mention
of the grant of the patent:
**24.12.2014 Bulletin 2014/52**

(51) Int Cl.:
***G01N 27/18*** *(2006.01)* ***G01N 33/22*** *(2006.01)*

(21) Application number: **13158709.9**

(22) Date of filing: **12.03.2013**

(54) **Electric power generating system and gas measuring system**

Stromerzeugungssystem und Gasmesssystem

Système de génération de puissance électrique et système de mesure de gaz

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.03.2012 JP 2012072203**

(43) Date of publication of application:
**02.10.2013 Bulletin 2013/40**

(73) Proprietor: **Azbil Corporation
Chiyoda-ku
Tokyo 100-6419 (JP)**

(72) Inventor: **Ooishi, Yasuharu
Tokyo, 100-6419 (JP)**

(74) Representative: **Klunker . Schmitt-Nilson . Hirsch
Patentanwälte
Destouchesstraße 68
80796 München (DE)**

(56) References cited:
**EP-A1- 0 529 295     EP-A1- 2 372 359
EP-A2- 2 369 337     US-A1- 2008 113 232**

**Description**

<u>Background</u>

The present invention relates to a gas inspection technology. Specifically, the present invention relates to an electric power generating system and a gas measuring system.

In recent years, a household power source in the spotlight is a fuel cell (for example, see Japanese Patent Application Laid-open No. H10-284104 and Japanese Patent Application Laid-open No. 2002-315224). One reason is that a fuel cell consumes less primary energy such as petroleum and natural gas. Another reason is that a fuel cell emits less carbon dioxide ($CO_2$). There are various types of fuel cells. One type of fuel cells is a polymer electrolyte fuel cell. A polymer electrolyte fuel cell includes a positive electrode, a negative electrode, and an ion-exchange membrane therebetween. The polymer electrolyte fuel cell supplies oxidant to the positive electrode, and supplies reductant (fuel) to the negative electrode. As a result, the polymer electrolyte fuel cell generates electric power. Hydrogen is used as the fuel. Hydrogen is obtained by reforming city gas.

<u>Summary</u>

As described above, hydrogen supplied to a polymer electrolyte fuel cell as fuel reacts with oxygen. As a result, the polymer electrolyte fuel cell generates electric power. In view of this, it is desirable to provide a technology capable of accurately measuring an amount of hydrogen supplied to the fuel cell. Further, it is desirable to provide a technology capable of accurately measuring atomic weight of atoms in molecules included in gas not only in the field of a fuel cell but also in a various technical fields. In view of the above-mentioned circumstances, it is an object of the present invention to provide an electric power generating system and a gas measuring system capable of estimating the number of atoms in molecules included in gas. According to an embodiment of the present invention, there is provided an electric power generating system, including: (a) a heating element configured to produce heat at a plurality of heating temperatures, the heating element being exposed to gas; (b) a measuring section configured to measure values of electric signals output from the heating element at the plurality of heating temperatures, respectively; (c) an equation storage device storing a first equation, the first equation including independent variables and a dependent variable, the independent variables being electric signals output from the heating element at the plurality of heating temperatures, respectively, the dependent variable being a sum of the product of the number of hydrogen atoms in each kind of molecules included in the gas, atomic weight of the hydrogen atoms, and a volume fraction of the each kind of molecules; (d) a calculating section configured to substitute values of electric signals output from the heating element in the independent variables of the first equation, respectively, to calculate the sum of the product of the number of hydrogen atoms in each kind of molecules included in the gas, atomic weight of the hydrogen atoms, and a volume fraction of the each kind of molecules; (e) a fuel cell configured to be supplied with hydrogen extracted from the gas; and (f) a controller configured to control a supply amount of the hydrogen supplied to the fuel cell based on the calculated sum of the product.

According to an embodiment of the present invention, there is provided a method of controlling an electric power generating system, including: (a) exposing a heating element to gas; (b) causing the heating element to produce heat at a plurality of heating temperatures; (c) measuring values of electric signals output from the heating element at the plurality of heating temperatures, respectively; (d) preparing a first equation, the first equation including independent variables and a dependent variable, the independent variables being electric signals output from the heating element at the plurality of heating temperatures, respectively, the dependent variable being a sum of the product of the number of hydrogen atoms in each kind of molecules included in the gas, atomic weight of the hydrogen atoms, and a volume fraction of the each kind of molecules; (e) substituting values of electric signals output from the heating element in the independent variables of the first equation, respectively, to calculate the sum of the product of the number of hydrogen atoms in each kind of molecules included in the gas, atomic weight of the hydrogen atoms, and a volume fraction of the each kind of molecules; and (f) controlling a supply amount of hydrogen supplied to the fuel cell based on the calculated sum of the product, the hydrogen being extracted from the gas.

According to an embodiment of the present invention, there is provided a gas measuring system, including: (a) a heating element configured to produce heat at a plurality of heating temperatures, the heating element being exposed to gas; (b) a measuring section configured to measure values of electric signals output from the heating element at the plurality of heating temperatures, respectively; (c) an equation storage device storing an equation, the equation including independent variables and a dependent variable, the independent variables being electric signals output from the heating element at the plurality of heating temperatures, respectively, the dependent variable being a sum of the product of the number of atoms in each kind of molecules included in the gas, atomic weight of the atoms, and a volume fraction of the each kind of molecules; and (d) a calculating section configured to substitute values of electric signals output from the heating element in the independent variables of the equation, respectively, to calculate the sum of the product of the number of atoms in each kind of molecules included in the gas, atomic weight of the atoms, and a volume fraction of the each kind of molecules.

According to an embodiment of the present invention, there is provided a gas measuring method, including: (a) exposing a heating element to gas; (b) causing the heating element to produce heat at a plurality of heating temperatures; (c) measuring values of electric signals output from the heating element at the plurality of heating temperatures, respectively; (d) preparing an equation, the equation including independent variables and a dependent variable, the independent variables being electric signals output from the heating element at the plurality of heating temperatures, respectively, the dependent variable being a sum of the product of the number of atoms in each kind of molecules included in the gas, atomic weight of the atoms, and a volume fraction of the each kind of molecules; and (e) substituting values of electric signals output from the heating element in the independent variables of the equation, respectively, to calculate the sum of the product of the number of atoms in each kind of molecules included in the gas, atomic weight of the atoms, and a volume fraction of the each kind of molecules.

According to the present invention, it is possible to provide an electric power generating system and a gas measuring system capable of estimating the number of atoms in molecules included in gas.

Brief Description of Drawings

Fig. 1 is a first perspective view showing a microchip according to a first embodiment of the present invention;

Fig. 2 is a sectional view of the microchip according to the first embodiment of the present invention, which is taken along the line II-II of Fig. 1;

Fig. 3 is a second perspective view showing a microchip according to the first embodiment of the present invention;

Fig. 4 is a sectional view of the microchip according to the first embodiment of the present invention, which is taken along the line IV-IV Fig. 3;

Fig. 5 is a circuit diagram showing a heating element according to the first embodiment of the present invention;

Fig. 6 is a circuit diagram showing a temperature detector according to the first embodiment of the present invention;

Fig. 7 is a graph showing relation between thermal conductivity and heat-radiation coefficients according to the embodiment of the present invention;

Fig. 8 is a graph showing relation between heat-radiation coefficients of gas and temperature of the heating element according to the first embodiment of the present invention;

Fig. 9 is a first graph showing relation between thermal conductivity and resistance of the heating element according to the embodiment of the present invention;

Fig. 10 is a second graph showing relation between thermal conductivity and resistance of the heating element according to the embodiment of the present invention;

Fig. 11 is a third graph showing relation between thermal conductivity and resistance of the heating element according to the embodiment of the present invention;

Fig. 12 is a fourth graph showing relation between thermal conductivity and resistance of the heating element according to the embodiment of the present invention;

Fig. 13 is a first graph showing relation between thermal conductivity and electric power for driving the heating element according to the embodiment of the present invention;

Fig. 14 is a second graph showing relation between thermal conductivity and electric power for driving the heating element according to the embodiment of the present invention;

Fig. 15 is a first schematic diagram showing a gas measuring system according to the first embodiment of the present invention;

Fig. 16 is a second schematic diagram showing the gas measuring system according to the first embodiment of the present invention;

Fig. 17 is a flowchart showing a method of creating an equation according to the first embodiment of the present invention;

Fig. 18 is a flowchart showing a gas measuring method according to the first embodiment of the present invention;

Fig. 19 is a graph showing errors, each of the errors being a difference between a measured value and a true value, according to a first example of the first embodiment of the present invention;

Fig. 20 is a graph showing errors, each of the errors being a difference between a measured value and a true value, according to a second example of the first embodiment of the present invention;

Fig. 21 is a graph showing errors, each of the errors being a difference between a measured value and a true value, according to an example of a second embodiment of the present invention; and

Fig. 22 is a schematic diagram showing an electric power generating system according to a fourth embodiment of the present invention.

Detailed Description of Embodiments

Hereinafter, embodiments of the present invention will be described with reference to the drawings. In the drawings, the same or similar parts are denoted by the same or similar reference symbols. Note that the drawings are schematic drawings. Specific dimensions and the like are to be determined with reference to the following description. As a matter of course, dimensional relation between one figure and another figure may be different, and a dimensional ratio between one figure and another figure may be different.

(First Embodiment)

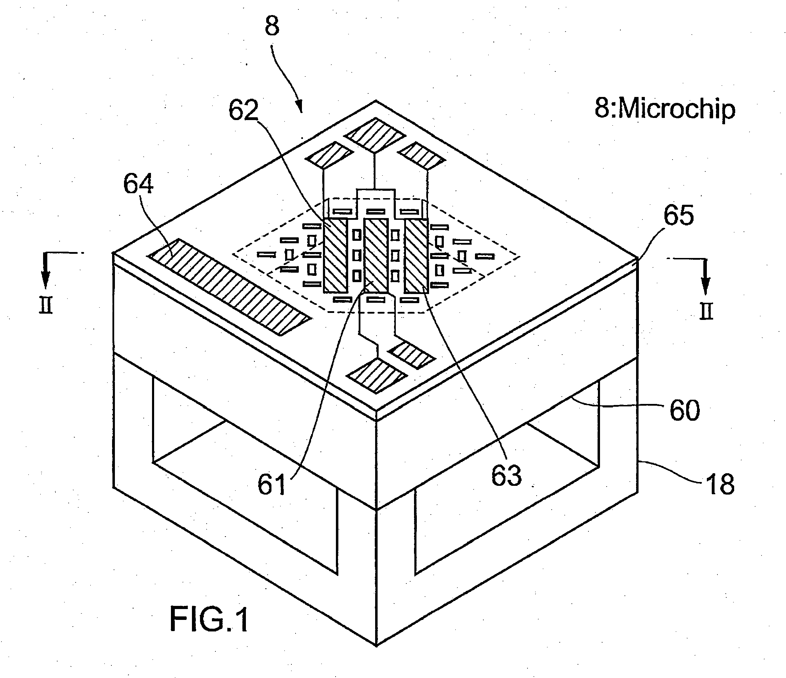

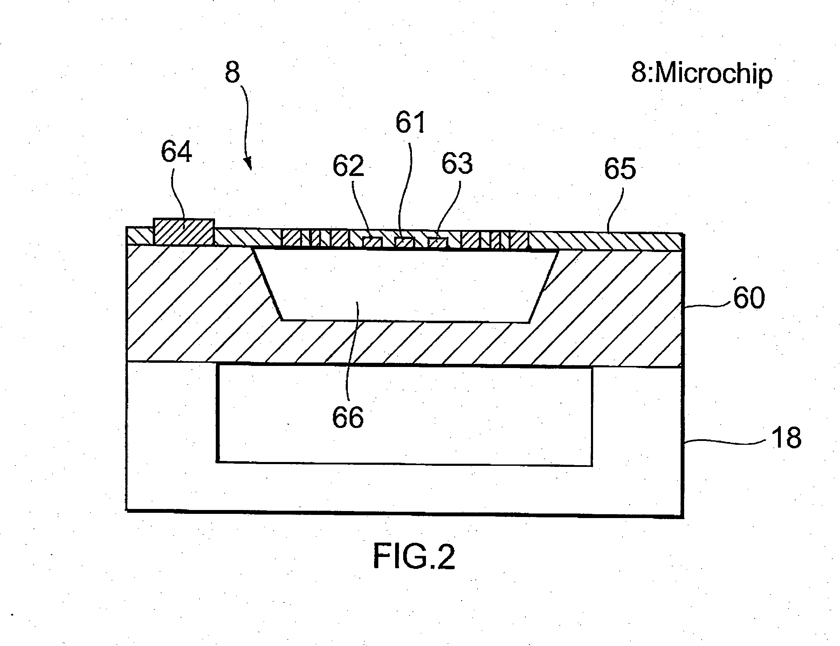

Fig. 1 is a perspective view showing a microchip 8. Fig. 2 is a sectional view of the microchip 8, which is taken along the line II-II. First, with reference to Fig. 1 and Fig. 2, the microchip 8 used in a gas measuring system according to a first embodiment will be described. The microchip 8 includes a substrate 60 and an insulating film 65. The substrate 60 has a cavity 66. The insulating film 65 is arranged on the substrate 60 such that the insulating film 65 covers the cavity 66. The thickness of the substrate 60 is, for example, 0.5 mm. Further, the length of the substrate 60 is, for example, about 1.5 mm. The width of the substrate 60 is, for example, about 1.5 mm. Part of the insulating film 65, which covers the cavity 66, is a thermalinsulating diaphragm. The microchip 8 further includes a heating element 61, a first temperature detector 62, a second temperature detector 63, and a heat-retention device 64. The heating element 61, the first temperature detector 62, and the second temperature detector 63 are provided in the diaphragm portion of the insulating film 65. The heating element 61 is arranged between the first temperature detector 62 and the second temperature detector 63. The heat-retention device 64 is provided on the substrate 60.

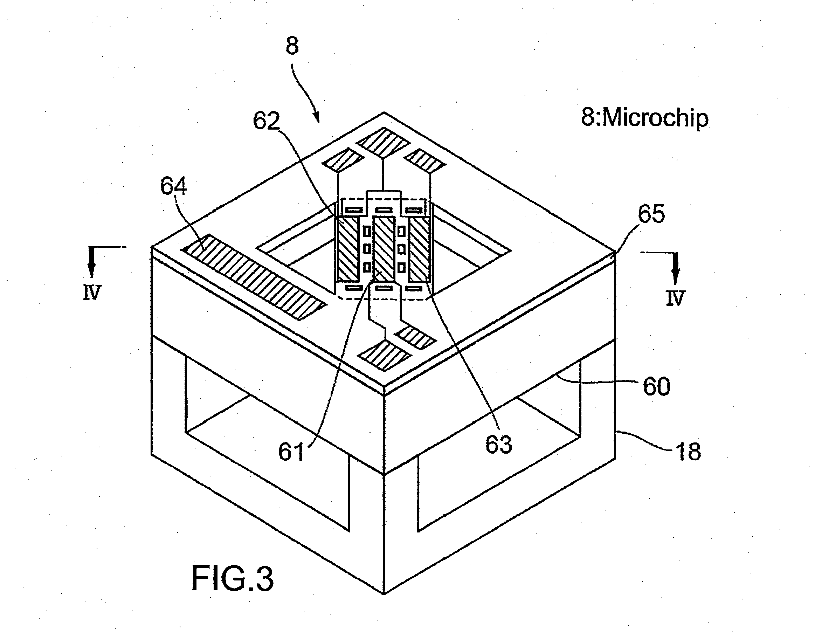

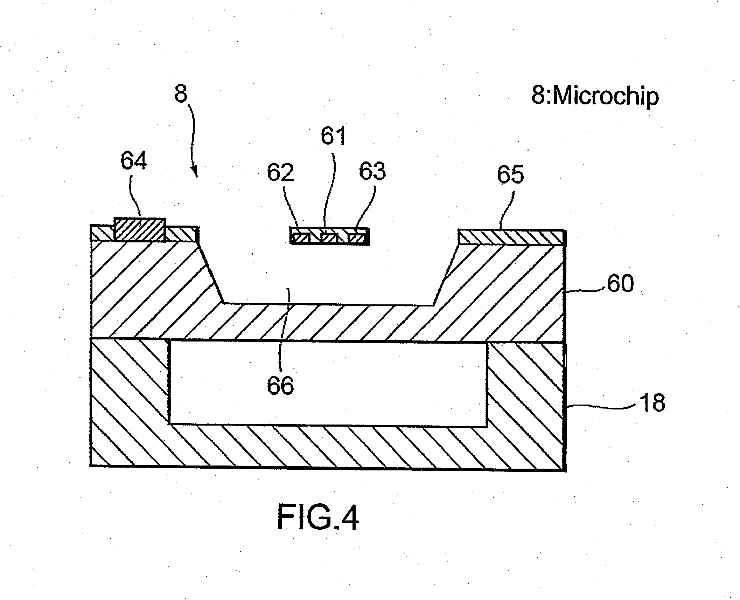

The diaphragm has a plurality of holes. Since the diaphragm has the plurality of holes, gas in the cavity 66 is exchanged rapidly. Alternatively, as shown in Fig. 3 and Fig. 4 (sectional view taken along the line IV-IV of Fig. 3), the insulating film 65 may be arranged on the substrate 60 such that the insulating film 65 covers the cavity 66 in a bridge-like manner. Part of the cavity 66 is thus exposed, and gas in the cavity 66 is exchanged rapidly.

The heating element 61 is arranged at the center of the diaphragm portion of the insulating film 65, which covers the cavity 66. The heating element 61 is, for example, a resistor. The heating element 61 receives electric power, produces heat, and heats the atmosphere gas exposed to the heating element 61. Each of the first temperature detector 62 and the second temperature detector 63 is, for example, an electronic device such as a passive device. An example of the passive device is a resistor. Each of the first temperature detector 62 and the second temperature detector 63 outputs an electric signal depending on the gas temperature of the atmosphere gas. Hereinafter, an example, in which a signal output from the first temperature detector 62 is used, will be described. However, this embodiment is not limited to this. For example, an average value of a signal output from the first temperature detector 62 and a signal output from the second temperature detector 63 may be used as a signal output from the temperature detectors.

The heat-retention device 64 is, for example, a resistor. The heat-retention device 64 receives electric power, produces heat, and keeps the temperature of the substrate 60 constant. The substrate 60 may be made from silicon (Si) or the like. The insulating film 65 may be made from silicon oxide ($SiO_2$) or the like. The cavity 66 is formed by means of anisotropic etching or the like. Further, each of the heating element 61, the first temperature detector 62, the second temperature detector 63, and the heat-retention device 64 may be made from platinum (Pt) or the like, and may be formed by means of lithography or the like. Further, the heating element 61, the first temperature detector 62, and the second temperature detector 63 may be the same components.

A heat-insulating member 18 is arranged on a bottom surface of the microchip 8. The microchip 8 is fixed on a pipe or the like, in which atmosphere gas flows, via the heat-insulating member 18. Since the microchip 8 is fixed on the pipe via the heat-insulating member 18, the temperature of the microchip 8 is unlikely to be affected by temperature fluctuation of an inner wall of the pipe. The heat-insulating member 18 is made from glass or the like. The thermal conductivity of the heat-insulating member 18 is, for example, 1.0 W/(m·K) or less.

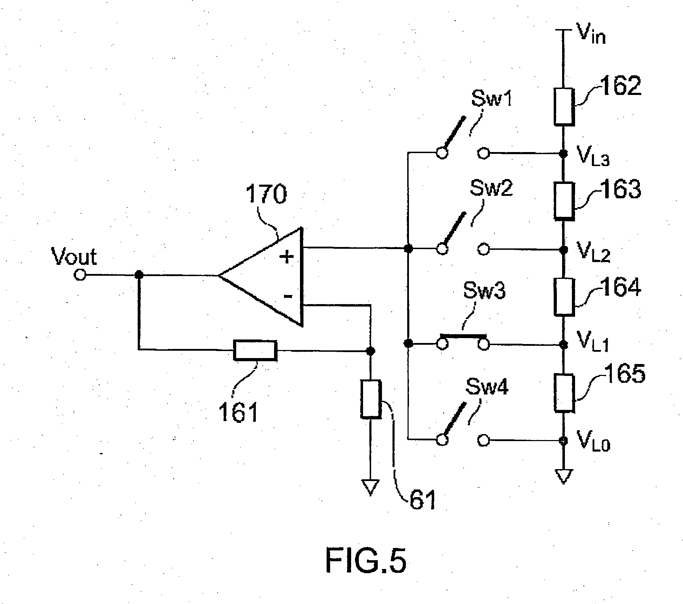

As shown in Fig. 5, for example, a - input terminal of an operational amplifier 170 is electrically connected to one end of the heating element 61. The other end of the heating element 61 is grounded. Further, the resistor 161 is connected in parallel with the - input terminal and an output terminal of the operational amplifier 170. A + input terminal of the operational amplifier 170 is electrically connected between a resistor 162 and a resistor 163, between the resistor 163 and a resistor 164, between the resistor 164 and a resistor 165, or to a grounded terminal of the resistor 165. The resistor 162, the resistor 163, the resistor 164, and the resistor 165 are connected in series. Each of the resistors 162 to 165 has a predetermined resistance value. A voltage $V_{in}$ is applied to one end of the resistor 162, for example. In this case, according to this structure, a first voltage $V_{L1}$ is generated between the resistor 165 and the resistor 164. A second voltage $V_{L2}$ is generated between the resistor 164 and the resistor 163. The second voltage $V_{L2}$ is higher than the first voltage $V_{L1}$. A third voltage $V_{L3}$ is generated between the resistor 163 and the resistor 162. The third voltage $V_{L3}$ is higher than the second voltage $V_{L2}$.

A switch Sw1 is provided between a line connecting the resistor 162 and the resistor 163, and the + input terminal of the operational amplifier. A switch Sw2 is provided between a line connecting the resistor 163 and the resistor 164, and the + input terminal of the operational amplifier. A switch Sw3 is provided between a line connecting the resistor 164 and the resistor 165, and the + input terminal of the operational amplifier. A switch Sw4 is provided between the grounded terminal of the resistor 165 and the + input terminal of the operational amplifier.

In a case where the third voltage $V_{L3}$ is applied to the + input terminal of the operational amplifier 170, a current passes only through the switch Sw1, and the switches Sw2, Sw3, and Sw4 are not connected. In a case where the second voltage $V_{L2}$ is applied to the + input terminal of the operational amplifier 170, a current passes only through the switch Sw2, and the switches Sw1, Sw3, and Sw4 are not connected. In a case where the first voltage $V_{L1}$ is applied to the + input terminal of the operational amplifier 170, a current passes only through the switch Sw3, and the switches Sw1, Sw2, and Sw4 are not connected. In a case where a voltage $V_{L0}$ (0 V) is applied to the + input terminal of the operational amplifier 170, a current passes only through the switch Sw4, and the switches Sw1, Sw2, and Sw3 are not connected. In this manner, it is possible to apply 0 V or one of the three levels of voltages to the + input terminal of the operational amplifier 170, by opening/closing the switches Sw1, Sw2, Sw3, and Sw4. That is, it is possible to set applied voltage, which determines a heating temperature of the heating element 61, at three levels, by opening/closing the switches Sw1, Sw2, Sw3, and Sw4.

Here, $T_{H1}$ is indicative of the temperature of the heating element 61 in a case where the first voltage $V_{L1}$ is applied to the + input terminal of the operational amplifier 170. $T_{H2}$ is indicative of the temperature of the heating element 61 in a case where the second voltage $V_{L2}$ is applied to the + input terminal of the operational amplifier 170. $T_{H3}$ is indicative of the temperature of the heating element 61 in a case where the third voltage $V_{L3}$ is applied to the + input terminal of the operational amplifier 170.

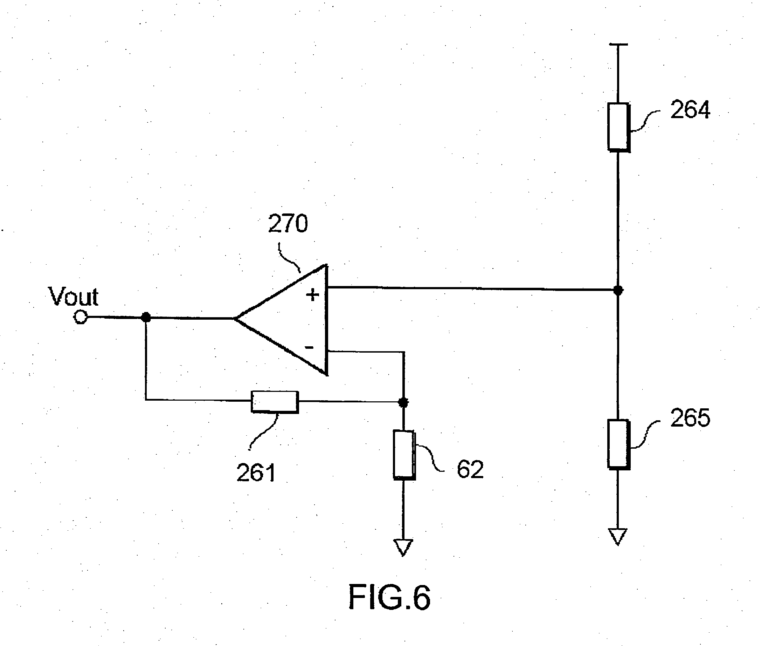

As shown in Fig. 6, for example, a - input terminal of an operational amplifier 270 is electrically connected to one end of the first temperature detector 62. The other end of the first temperature detector 62 is grounded. Further, a resistor 261 is connected in parallel with the - input terminal and an output terminal of the operational amplifier 270. A + input terminal of the operational amplifier 270 is electrically connected to a line connecting a resistor 264 and a resistor 265. The resistor 264 and the resistor 265 are connected in series. According to this structure, a small voltage (about 0.3 V) is applied to the first temperature detector 62.

The resistance value of the heating element 61 of Fig. 1 and Fig. 2 depends on the temperature of the heating element 61. The following Equation (1) shows the relation between the temperature $T_H$ of the heating element 61 and the resistance value $R_H$ of the heating element 61.

$$R_H = R_{H\_STD} \times [1 + \alpha_H (T_H - T_{H\_STD}) + \beta_H (T_H - T_{H\_STD})^2] \quad \ldots (1)$$

Here, $T_{H\_STD}$ is indicative of a standard temperature of the heating element 61, and is, for example 20°C. $R_{H\_STD}$ is indicative of a previously-measured resistance value of the heating element 61 at the standard temperature $T_{H\_STD}$. $\alpha_H$ is indicative of a primary resistance temperature coefficient. $\beta_H$ is indicative of a secondary resistance temperature coefficient.

As shown in the following Equation (2), the resistance value $R_H$ of the heating element 61 is obtained based on the electric power $P_H$ for driving the heating element 61 and based on the current $I_H$ passing through the heating element 61.

$$R_H = P_H / I_H^2 \quad \ldots (2)$$

Alternatively, as shown in the following Equation (3), the resistance value $R_H$ of the heating element 61 is obtained based on the voltage $V_H$ applied to the heating element 61 and based on the current $I_H$ passing through the heating element 61.

$$R_H = V_H / I_H \quad \ldots (3)$$

Here, the temperature $T_H$ of the heating element 61 is stable when the heating element 61 and the atmosphere gas are thermally balanced. Note that the thermally balanced status means a status in which heat produced by the heating element 61 and heat radiated from the heating element 61 to the atmosphere gas are balanced. As shown in the Equation (4), the electric power $P_H$ for driving the heating element 61 under the balanced status is divided by $\Delta T_H$. $\Delta T_H$ is a difference between the temperature $T_H$ of the heating element 61 and the temperature $T_I$ of the atmosphere gas. As a result, the heat-radiation coefficient $M_I$ (e.g., W/°C) of the atmosphere gas is obtained.

$$M_I = P_H / (T_H - T_I)$$
$$= P_H / \Delta T_H = (V_H{}^2 / R_H) / \Delta T_H \quad \dots (4)$$

Based on Equation (1), the following Equation (5) shows the temperature $T_H$ of the heating element 61.

$$T_H = (1/2\beta_H) \times [-\alpha_H + [\alpha_H{}^2 - 4\beta_H (1 - R_H / R_{H\_STD})]^{1/2}] + T_{H\_STD} \quad \dots (5)$$

In view of this, the following Equation (6) shows the difference $\Delta T_H$ between the temperature $T_H$ of the heating element 61 and the temperature $T_I$ of the atmosphere gas.

$$\Delta T_H = (1/2\beta_H) \times [-\alpha_H + [\alpha_H{}^2 - 4\beta_H (1 - R_H / R_{H\_STD})]^{1/2}] + T_{H\_STD} - T_I \quad \dots (6)$$

The temperature $T_I$ of the atmosphere gas is approximate to the temperature $T_I$ of the first temperature detector 62. Electric power is supplied to the first temperature detector 62 such that the first temperature detector 62 does not produce heat by itself. The following Equation (7) shows the relation between the temperature $T_I$ of the first temperature detector 62 and the resistance value $R_I$ of the first temperature detector 62.

$$R_I = R_{I\_STD} \times [1 + \alpha_I (T_I - T_{I\_STD}) + \beta_I (T_I - T_{I\_STD})^2] \quad \dots (7)$$

$T_{I\_STD}$ is indicative of a standard temperature of the first temperature detector 62 and is, for example, 20°C. $R_{I\_STD}$ is indicative of the previously-measured resistance value of the first temperature detector 62 at the standard temperature $T_{I\_STD}$. $\alpha_I$ is indicative of a primary resistance temperature coefficient. $\beta_I$ is indicative of a secondary resistance temperature coefficient. In view of Equation (7), the following Equation (8) shows the temperature $T_I$ of the first temperature detector 62.

$$T_I = (1/2\beta_I) \times [-\alpha_I + [\alpha_I{}^2 - 4\beta_I (1 - R_I / R_{I\_STD})]^{1/2}] + T_{I\_STD} \quad \dots (8)$$

Accordingly, the following Equation (9) shows the heat-radiation coefficient $M_I$ of the atmosphere gas.

$$M_I = P_H / \Delta T_H$$
$$= P_H / [(1/2\beta_H) [-\alpha_H + [\alpha_H{}^2 - 4\beta_H (1 - R_H / R_{H\_STD})]^{1/2}] + T_{H\_STD} - (1/2\beta_I) [-\alpha_I + [\alpha_I{}^2 -$$
$$4\beta_I (1 - R_I / R_{I\_STD})]^{1/2}] - T_{I\_STD}] \quad \dots (9)$$

It is possible to measure the current $I_H$ passing through the heating element 61 and the electric power $P_H$ for driving the heating element 61 or the voltage $V_H$ applied to the heating element 61. Therefore it is possible to calculate the resistance value $R_H$ of the heating element 61 by using Equation (2) or Equation (3). Similarly, it is possible to calculate the resistance value $R_I$ of the first temperature detector 62. Therefore, with the microchip 8, it is possible to calculate the heat-radiation coefficient $M_I$ of the atmosphere gas based on Equation (9).

Note that the heat-retention device 64 keeps the temperature of the substrate 60 constant. As a result, before the heating element 61 produces heat, the temperature of the atmosphere gas around the microchip 8 is approximate to the constant temperature of the substrate 60. Because of this, fluctuation of the temperature of the atmosphere gas is reduced before the heating element 61 produces heat. The heating element 61 further heats the atmosphere gas, whose temperature fluctuation is once reduced. As a result, it is possible to calculate the heat-radiation coefficient $M_I$ more accurately.

Here, the atmosphere gas is mixed gas including four gas components, i.e., gas A, gas B, gas C, and gas D. As shown in the following Equation (10), the sum of the volume fraction $V_A$ of the gas A, the volume fraction $V_B$ of the gas B, the volume fraction $V_C$ of the gas C, and the volume fraction $V_D$ of the gas D is 1.

$$V_A + V_B + V_C + V_D = 1 \quad \ldots (10)$$

$K_A$ is indicative of the calorific value of the gas A per unit volume. $K_B$ is indicative of the calorific value of the gas B per unit volume. $K_C$ is indicative of the calorific value of the gas C per unit volume. $K_D$ is indicative of the calorific value of the gas D per unit volume. Q is indicative of the calorific value of the mixed gas per unit volume. In this case, Q equals to the sum of values, which are obtained by multiplying the volume fractions of the gas components by the calorific values of heat produced by the gas components per unit volume, respectively. That is, the following Equation (11) shows the calorific value Q (e.g., $MJ/m^3$) of the mixed gas per unit volume.

$$Q = K_A \times V_A + K_B \times V_B + K_C \times V_C + K_D \times V_D \quad \ldots (11)$$

Further, $K_{AH}$ is indicative of the calorific value produced by hydrogen atoms in the gas A, per unit volume. $K_{BH}$ is indicative of the calorific value produced by hydrogen atoms in the gas B, per unit volume. $K_{CH}$ is indicative of the calorific value produced by hydrogen atoms in the gas C, per unit volume. $K_{DH}$ is indicative of the calorific value produced by hydrogen atoms in the gas D, per unit volume. $Q_H$ is indicative of the calorific value produced by hydrogen atoms in molecules in the mixed gas, per unit volume. In this case, $Q_H$ equals to the sum of the values, which are obtained by multiplying the volume fractions of the gas components by the calorific values produced by hydrogen atoms in the gas components, per unit volume, respectively. That is, the following Equation (12) shows the calorific value $Q_H$ produced by hydrogen atoms in molecules in the mixed gas, per unit volume.

$$Q_H = K_{AH} \times V_A + K_{BH} \times V_B + K_{CH} \times V_C + K_{DH} \times V_D \quad \ldots (12)$$

Next, $C_A$ is indicative of the thermal conductivity of the gas A per unit volume. $C_B$ is indicative of the thermal conductivity of the gas B per unit volume. $C_C$ is indicative of the thermal conductivity of the gas C per unit volume. $C_D$ is indicative of the thermal conductivity of the gas D per unit volume. $C_I$ is indicative of the thermal conductivity of the mixed gas per unit volume. In this case, $C_I$ equals to the sum of values, which are obtained by multiplying the volume fractions of the gas components by the thermal conductivities of the gas components per unit volume, respectively. That is, the following Equation (13) shows the thermal conductivity $C_I$ (e.g., W/(mK)) of the mixed gas per unit volume.

$$C_I = C_A \times V_A + C_B \times V_B + C_C \times V_C + C_D \times V_D \quad \ldots (13)$$

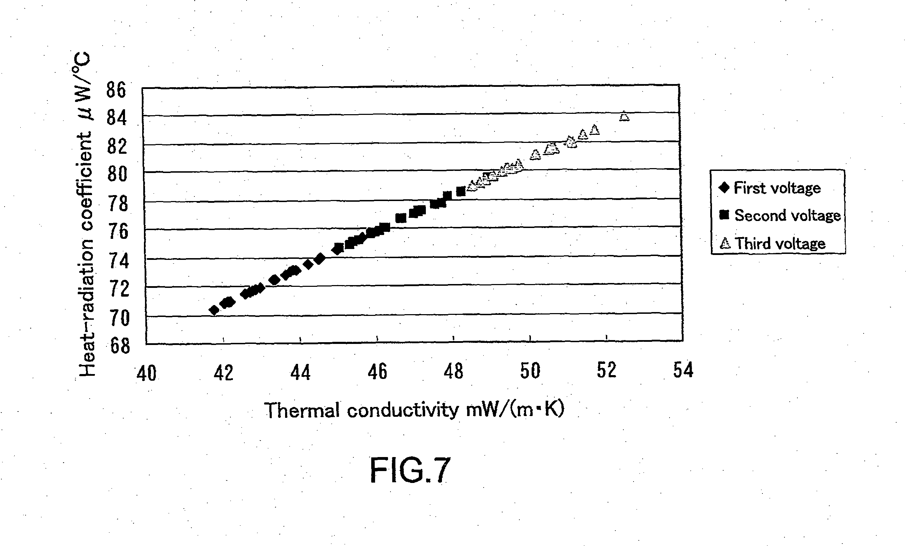

Fig. 7 is a graph showing the relation between the thermal conductivity and the heat-radiation coefficient. Fig. 7 shows that the first voltage $V_1$, the second voltage $V_2$, and the third voltage $V_3$ are applied to the heating element 61. The second voltage $V_2$ is higher than the first voltage $V_1$. The third voltage $V_3$ is higher than the second voltage $V_2$. As shown in Fig. 7, the thermal conductivity is in proportion to the heat-radiation coefficient, in general. $M_A$ is indicative of the heat-radiation coefficient of the gas A. $M_B$ is indicative of the heat-radiation coefficient of the gas B. $M_C$ is indicative of the heat-radiation coefficient of the gas C. $M_D$ is indicative of the heat-radiation coefficient of the gas D. $M_I$ is indicative of the heat-radiation coefficient of the mixed gas. In this case, $M_I$ equals to the sum of values, which are obtained by multiplying the volume fractions of the gas components by the heat-radiation coefficients of the gas components, respectively. That is, the following Equation (14) shows the heat-radiation coefficient $M_I$ of the mixed gas.

$$M_I = M_A \times V_A + M_B \times V_B + M_C \times V_C + M_D \times V_D \quad \ldots (14)$$

Further, the heat-radiation coefficient of gas depends on the temperature $T_H$ of the heating element 61. So the following Equation (15) shows the heat-radiation coefficient $M_I$ of the mixed gas, as a function of the temperature $T_H$ of the heating element 61.

$$M_I(T_H) = M_A(T_H) \times V_A + M_B(T_H) \times V_B + M_C(T_H) \times V_C + M_D(T_H) \times V_D \quad \ldots (15)$$

That is, the following Equation (16) shows the heat-radiation coefficient $M_{I1}$ of the mixed gas ($T_{H1}$) where $T_{H1}$ is indicative of the temperature of the heating element 61. The following Equation (17) shows the heat-radiation coefficient

$M_{I2}$ of the mixed gas ($T_{H2}$) where $T_{H2}$ is indicative of the temperature of the heating element 61. The following Equation (18) shows the heat-radiation coefficient $M_{I3}$ of the mixed gas ($T_{H3}$) where $T_{H3}$ is indicative of the temperature of the heating element 61.

$$M_{I1}(T_{H1}) = M_A(T_{H1}) \times V_A + M_B(T_{H1}) \times V_B + M_C(T_{H1}) \times V_C + M_D(T_{H1}) \times V_D \quad \ldots (16)$$

$$M_{I2}(T_{H2}) = M_A(T_{H2}) \times V_A + M_B(T_{H2}) \times V_B + M_C(T_{H2}) \times V_C + M_D(T_{H2}) \times V_D \quad \ldots (17)$$

$$M_{I3}(T_{H3}) = M_A(T_{H3}) \times V_A + M_B(T_{H3}) \times V_B + M_C(T_{H3}) \times V_C + M_D(T_{H3}) \times V_D \quad \ldots (18)$$

Here, the heat-radiation coefficients $M_A(T_H)$, $M_B(T_H)$, $M_C(T_H)$, $M_D(T_H)$ of the respective gas components are non-linear with respect to the temperature $T_H$ of the heating element 61. In this case, each of Equations (16) to (18) has a linear-independent relation. Further, the heat-radiation coefficients $M_A(T_H)$, $M_B(T_H)$, $M_C(T_H)$, $M_D(T_H)$ of the respective gas components are linear with respect to the temperature $T_H$ of the heating element 61. In addition, change rates of the heat-radiation coefficients $M_A(T_H)$, $M_B(T_H)$, $M_C(T_H)$, $M_D(T_H)$ of the respective gas components with respect to the temperature $T_H$ of the heating element 61 are different from each other. Also in this case, each of Equations (16) to (18) has a linear-independent relation. Further, in a case where each of Equations (16) to (18) has a linear-independent relation, each of Equation (10) and Equations (16) to (18) has a linear-independent relation.

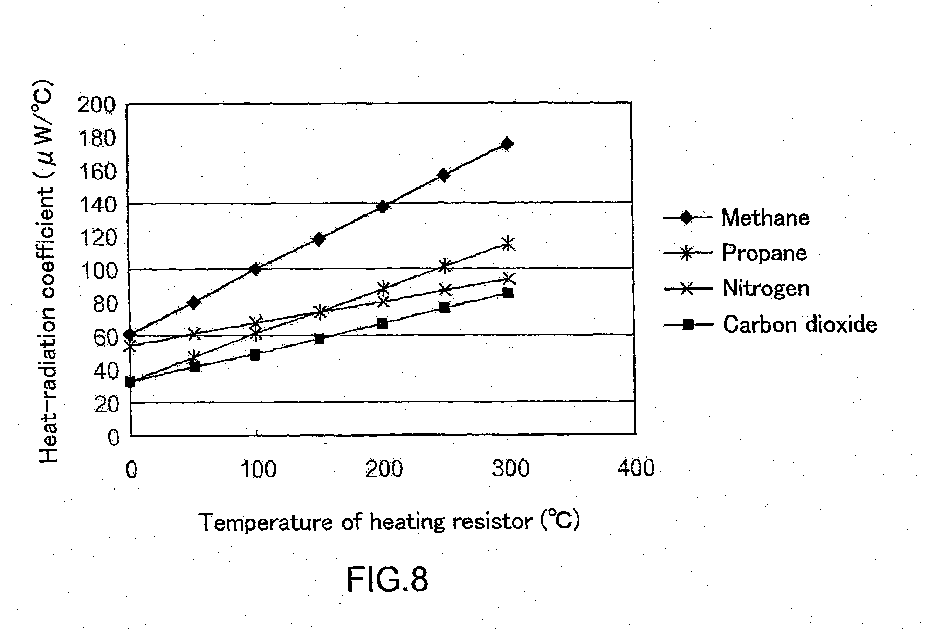

Fig. 8 is a graph showing relation between heat-radiation coefficients of methane ($CH_4$), propane ($C_3H_8$), nitrogen ($N_2$), and carbon dioxide ($CO_2$) and the temperature of the heating element 61. Methane ($CH_4$), propane ($C_3H_8$), nitrogen ($N_2$), and carbon dioxide ($CO_2$) are included in natural gas or city gas. The heating element 61 is a heating resistor. The heat-radiation coefficient of each of the gas components (methane ($CH_4$), propane ($C_3H_8$), nitrogen ($N_2$), and carbon dioxide ($CO_2$)) is linear with respect to the temperature of the heating element 61. However, the change rates of the heat-radiation coefficients of methane ($CH_4$), propane ($C_3H_8$), nitrogen ($N_2$), and carbon dioxide ($CO_2$) with respect to the temperature of the heating element 61 are different from each other. In view of this, in the case where the mixed gas includes methane ($CH_4$), propane ($C_3H_8$), nitrogen ($N_2$), and carbon dioxide ($CO_2$) as gas components, each of Equations (16) to (18) has a linear-independent relation.

The heat-radiation coefficients $M_A(T_{H1})$, $M_B(T_{H1})$, $M_C(T_{H1})$, $M_D(T_{H1})$, $M_A(T_{H2})$, $M_B(T_{H2})$, $M_C(T_{H2})$, $M_D(T_{H2})$, $M_A(T_{H3})$, $M_B(T_{H3})$, $M_C(T_{H3})$, $M_D(T_{H3})$ of the gas components of Equations (16) to (18) may be previously obtained by measuring or the like. In view of this, by solving a simultaneous equation including Equation (10) and Equations (16) to (18), the volume fraction $V_A$ of the gas A, the volume fraction $V_B$ of the gas B, the volume fraction $V_C$ of the gas C, and the volume fraction $V_D$ of the gas D are obtained. As shown in each of the following Equations (19) to (22), each of the volume fraction $V_A$ of the gas A, the volume fraction $V_B$ of the gas B, the volume fraction $V_C$ of the gas C, and the volume fraction $V_D$ of the gas D is obtained as a function of the heat-radiation coefficients $M_{I1}(T_{H1})$, $M_{I2}(T_{H2})$, $M_{I3}(T_{H3})$ of the mixed gas. Note that, in the following Equations (19) to (22), n is indicative of a natural number, and $f_n$ is indicative of a function.

$$V_A = f_1[M_{I1}(T_{H1}), M_{I2}(T_{H2}), M_{I3}(T_{H3})] \quad \ldots (19)$$

$$V_B = f_2[M_{I1}(T_{H1}), M_{I2}(T_{H2}), M_{I3}(T_{H3})] \quad \ldots (20)$$

$$V_C = f_3[M_{I1}(T_{H1}), M_{I2}(T_{H2}), M_{I3}(T_{H3})] \quad \ldots (21)$$

$$V_D = f_4[M_{I1}(T_{H1}), M_{I2}(T_{H2}), M_{I3}(T_{H3})] \quad \ldots (22)$$

Here, Equations (19) to (22) are substituted in Equation (11). As a result, the following Equation (23) is obtained.

$$Q=K_A \times V_A+K_B \times V_B+K_C \times V_C+K_D \times V_D$$
$$=K_A \times f_1[M_{I1}(T_{H1}), \ M_{I2}(T_{H2}), \ M_{I3}(T_{H3})]$$
$$+K_B \times f_2[M_{I1}(T_{H1}), \ M_{I2}(T_{H2}), \ M_{I3}(T_{H3})]$$
$$+K_C \times f_3[M_{I1}(T_{H1}), \ M_{I2}(T_{H2}), \ M_{I3}(T_{H3})]$$
$$+K_D \times f_4[M_{I1}(T_{H1}), \ M_{I2}(T_{H2}), \ M_{I3}(T_{H3})] \ \ldots(23)$$

As shown in Equation (23), the calorific value Q of the mixed gas per unit volume is obtained based on an equation in which the heat-radiation coefficients $M_{I1}(T_{H1})$, $M_{I2}(T_{H2})$, $M_{I3}(T_{H3})$ of the mixed gas are variables. The heat-radiation coefficients $M_{I1}(T_{H1})$, $M_{I2}(T_{H2})$, $M_{I3}(T_{H3})$ of the mixed gas are values in the case where the temperatures of the heating element 61 are $T_{H1}$, $T_{H2}$, and $T_{H3}$, respectively. In view of this, the following Equation (24) shows the calorific value Q of the mixed gas. $g_1$ is indicative of a function.

$$Q=g_1[M_{I1}(T_{H1}), \ M_{I2}(T_{H2}), \ M_{I3}(T_{H3})] \ \ldots(24)$$

Further, the following Equation (25) is obtained according to Equation (12) and Equations (19) to (22). Equation (25) shows the calorific value $Q_H$ produced by hydrogen atoms in molecules in the mixed gas, per unit volume. $g_2$ is indicative of a function.

$$Q_H=g_2[M_{I1}(T_{H1}), \ M_{I2}(T_{H2}), \ M_{I3}(T_{H3})] \ \ldots(25)$$

In view of this, the inventors have found out the following fact. That is, it is possible to easily calculate the calorific value $Q_H$ produced by hydrogen atoms in molecules in the measuring-target mixed gas, per unit volume, by previously obtaining Equation (25) about the mixed gas including the gas A, the gas B, the gas C, and the gas D, even if the volume fraction $V_A$ of the gas A, the volume fraction $V_B$ of the gas B, the volume fraction $V_C$ of the gas C, and the volume fraction $V_D$ of the gas D are unknown. Specifically, the heat-radiation coefficients $M_{I1}(T_{H1})$, $M_{I2}(T_{H2})$, $M_{I3}(T_{H3})$ of the measuring-target mixed gas in the case where the heating temperatures of the heating element 61 are $T_{H1}$, $T_{H2}$, and $T_{H3}$, respectively, are measured. The measured $M_{I1}(T_{H1})$, $M_{I2}(T_{H2})$, $M_{I3}(T_{H3})$ are substituted in Equation (25). As a result, it is possible to uniquely obtain the calorific value $Q_H$ produced by hydrogen atoms in molecules in the measuring-target mixed gas, per unit volume.

Next, as shown in Equation (9), the heat-radiation coefficient $M_I$ of the mixed gas depends on the resistance value $R_H$ of the heating element 61 and on the resistance value $R_I$ of the first temperature detector 62. Then, the inventors of the present invention have found out that the calorific value $Q_H$ produced by hydrogen atoms in molecules in the mixed gas, per unit volume, is also obtained based on the following Equation (26). In Equation (26), $h_1$ is indicative of a function. The resistance values $R_{H1}(T_{H1})$, $R_{H2}(T_{H2})$, $R_{H3}(T_{H3})$ of the heating element 61 in the case where the temperatures of the heating element 61 are $T_{H1}$, $T_{H2}$, and $T_{H3}$, respectively, are variables. The resistance value $R_I$ of the first temperature detector 62 exposed to the mixed gas is a variable.

$$Q_H=h_1[R_{H1}(T_{H1}), \ R_{H2}(T_{H2}), \ R_{H3}(T_{H3}), \ R_I] \ \ldots(26)$$

In view of this, the resistance values $R_{H1}(T_{H1})$, $R_{H2}(T_{H2})$, $R_{H3}(T_{H3})$ of the heating element 61 in a case where the heating temperatures of the heating element 61 exposed to the measuring-target mixed gas are $T_{H1}$, $T_{H2}$, and $T_{H3}$, respectively, are measured. The resistance value $R_I$ of the first temperature detector 62 exposed to the measuring-target mixed gas is measured. The measured $R_{H1}(T_{H1})$, $R_{H2}(T_{H2})$, $R_{H3}(T_{H3})$, and $R_I$ are substituted in Equation (26). As a result, it is also possible to uniquely obtain the calorific value $Q_H$ produced by hydrogen atoms in molecules in the measuring-target mixed gas, per unit volume.

Further, the calorific value $Q_H$ produced by hydrogen atoms in molecules in the mixed gas, per unit volume, is also obtained based on the following Equation (27). In Equation (27), $h_2$ is indicative of a function. The currents $I_{H1}(T_{H1})$, $I_{H2}(T_{H2})$, $I_{H3}(T_{H3})$ passing through the heating element 61 in the case where the temperatures of the heating element 61 are $T_{H1}$, $T_{H2}$, and $T_{H3}$, respectively, are variables. The current $I_I$ of the first temperature detector 62 exposed to the mixed gas is a variable.

$$Q_H=h_2[I_{H1}(T_{H1}), I_{H2}(T_{H2}), I_{H3}(T_{H3}), I_I] \ldots(27)$$

Alternatively, the calorific value $Q_H$ produced by hydrogen atoms in molecules in the mixed gas, per unit volume, is also obtained based on the following Equation (28). In Equation (28), $h_3$ is indicative of a function. The voltages $V_{H1}(T_{H1})$, $V_{H2}(T_{H2})$, $V_{H3}(T_{H3})$ applied to the heating element 61 in the case where the temperatures of the heating element 61 are $T_{H1}$, $T_{H2}$, and $T_{H3}$, respectively, are variables. The voltage $V_I$ applied to the first temperature detector 62 exposed to the mixed gas is a variable.

$$Q_H=h_3[V_{H1}(T_{H1}), V_{H2}(T_{H2}), V_{H3}(T_{H3}), V_I] \ldots(28)$$

Alternatively, the calorific value $Q_H$ produced by hydrogen atoms in molecules in the mixed gas, per unit volume, is also obtained based on the following Equation (29). In Equation (29), $h_4$ is indicative of a function. The output signals $AD_{H1}(T_{H1})$, $AD_{H2}(T_{H2})$, $AD_{H3}(T_{H3})$ output from an analog-digital converter circuit (hereinafter referred to as "A/D converter circuit") connected to the heating element 61 in the case where the temperatures of the heating element 61 are $T_{H1}$, $T_{H2}$, and $T_{H3}$, respectively, are variables. The output signal $AD_I$ of an A/D converter circuit connected to the first temperature detector 62 exposed to the mixed gas is a variable.

$$Q_H=h_4[AD_{H1}(T_{H1}), AD_{H2}(T_{H2}), AD_{H3}(T_{H3}), AD_I] \ldots(29)$$

In view of this, the calorific value $Q_H$ produced by hydrogen atoms in molecules in the mixed gas, per unit volume, is obtained based on the following Equation (30). In Equation (30), $h_5$ is indicative of a function. The electric signals $S_{H1}(T_{H1})$, $S_{H2}(T_{H2})$, $S_{H3}(T_{H3})$ output from the heating element 61 in the case where the temperatures of the heating element 61 are $T_{H1}$, $T_{H2}$, and $T_{H3}$, respectively, are variables. The electric signal $S_I$ output from the first temperature detector 62 exposed to the mixed gas is a variable.

$$Q_H=h_5[S_{H1}(T_{H1}), S_{H2}(T_{H2}), S_{H3}(T_{H3}), S_I] \ldots(30)$$

According to the above-mentioned method, the calorific value $Q_H$ produced by hydrogen atoms in molecules in the mixed gas, per unit volume, is obtained by using the heating element 61 and the first temperature detector 62 of the microchip 8. Meanwhile, according to the following method, it is possible to obtain the calorific value $Q_H$ produced by hydrogen atoms in molecules in the mixed gas, per unit volume, only by using the heating element 61, without using the first temperature detector 62 of the microchip 8.

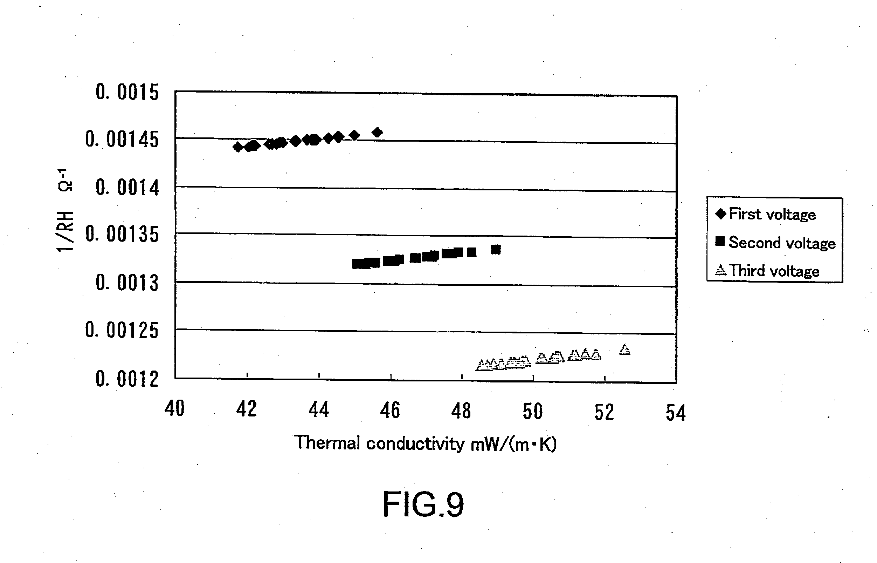

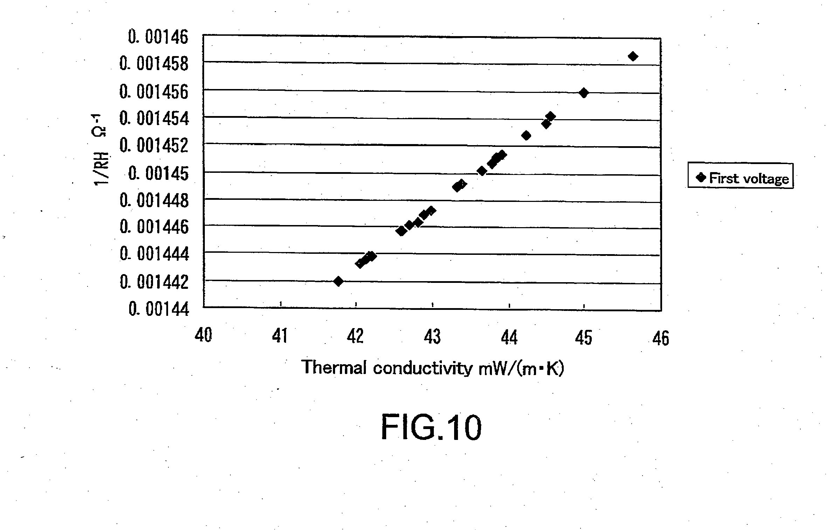

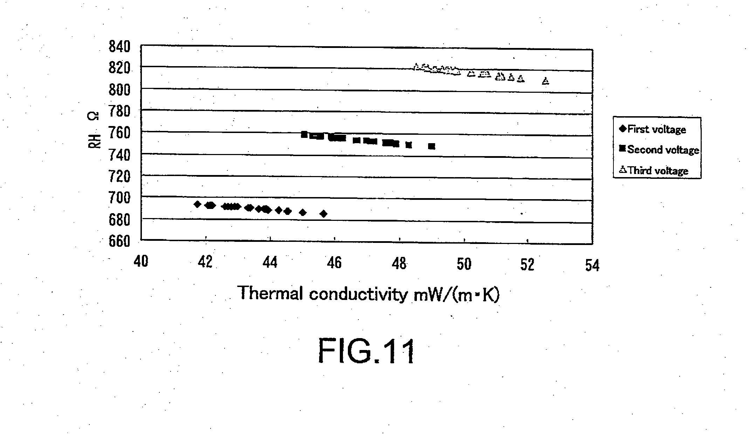

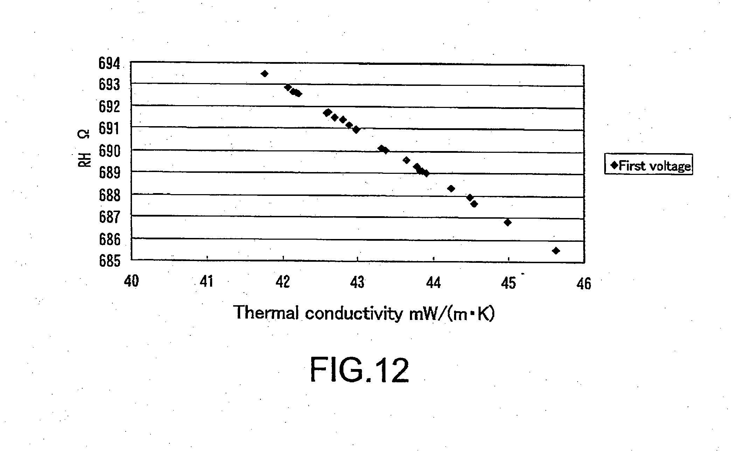

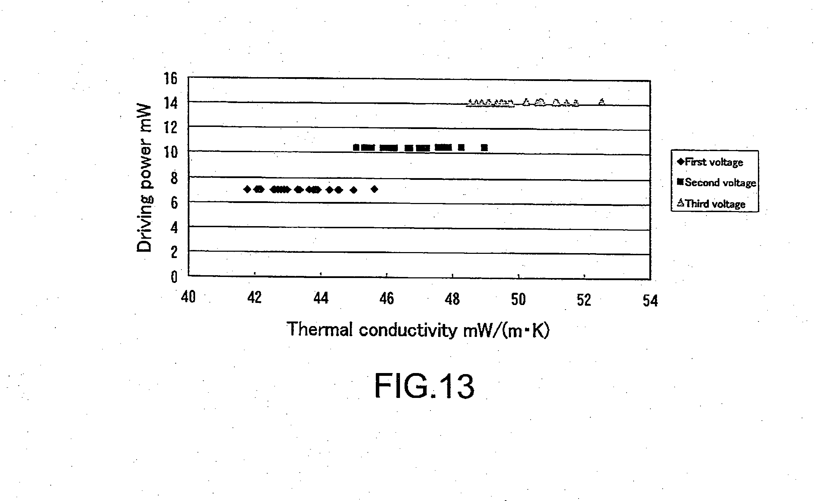

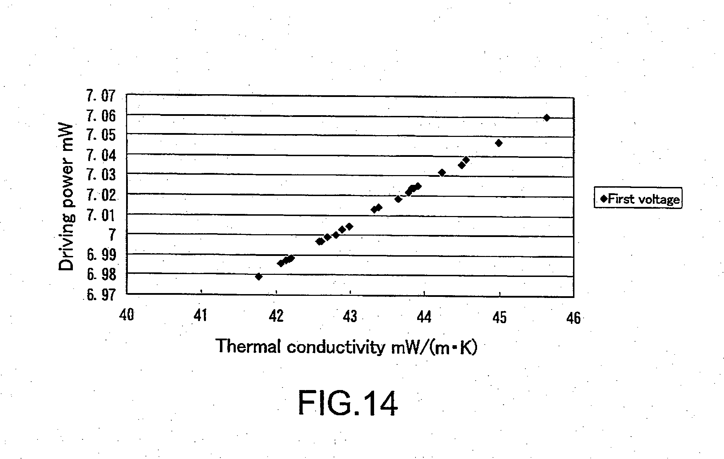

As shown in Equation (4), the heat-radiation coefficient $M_I$ of gas is in proportion to $1/R_H$. $1/R_H$ is the inverse number of the resistance value $R_H$ of the heating element 61. Further, as described above, the heat-radiation coefficient is in proportion to the thermal conductivity. As a result, the inverse number ($1/R_H$) of the resistance value $R_H$ of the heating element 61 is in proportion to the thermal conductivity. Fig. 9 is a graph showing relation between the thermal conductivity and the inverse number ($1/R_H$) of the resistance value $R_H$ of the heating element 61. In Fig. 9, the first voltage $V_1$, the second voltage $V_2$, and the third voltage $V_3$ are applied to the heating element 61. As shown in Fig. 9 and Fig. 10, the thermal conductivity is in proportion to the inverse number ($1/R_H$) of the resistance value $R_H$ of the heating element 61, if the voltage applied to the heating element 61 is constant. Further, as shown in Fig. 11 and Fig. 12, the thermal conductivity is in correlation with the resistance value $R_H$ of the heating element 61 if the voltage applied to the heating element 61 is constant. Further, as shown in Fig. 13 and Fig. 14, the thermal conductivity is in correlation with the electric power for driving the heating element 61, if the voltage applied to the heating element 61 is constant.

In view of this, $1/R_{HA}$ is indicative of the inverse number of the resistance value $R_H$ of the heating element 61 in a case where the heating element 61 is exposed to the gas A. $1/R_{HB}$ is indicative of the inverse number of the resistance value $R_H$ of the heating element 61 in a case where the heating element 61 is exposed to the gas B. $1/R_{HC}$ is indicative of the inverse number of the resistance value $R_H$ of the heating element 61 in a case where the heating element 61 is exposed to the gas C. $1/R_{HD}$ is indicative of the inverse number of the resistance value $R_H$ of the heating element 61 in a case where the heating element 61 is exposed to the gas D. In this case, the inverse number ($1/R_{HI}$) of the resistance value $R_H$ of the heating element 61 exposed to the mixed gas is obtained by modifying Equation (13). That is, $1/R_{HI}$ equals to the sum of values, which are obtained by multiplying the volume fractions of the gas components by the inverse numbers of the resistance values $R_H$ in a case where the heating element 61 is exposed to the gas components, respectively. As a result, the following Equation (31) shows the inverse number ($1/R_{HI}$) of the resistance value $R_H$ of the heating element 61 exposed to the mixed gas to which a constant voltage is applied.

$$1/R_{HI}=1/R_{HA}\times V_A+1/R_{HB}\times V_B+1/R_{HC}\times V_C+1/R_{HD}\times V_D \quad \cdots (31)$$

Further, the resistance value $R_H$ of the heating element 61 depends on the temperature $T_H$ of the heating element 61. So the inverse number ($1/R_{HI}$) of the resistance value $R_H$ of the heating element 61 exposed to the mixed gas is obtained based on the following Equation (32) as a function of the temperature $T_H$ of the heating element 61.

$$1/R_{HI}(T_H)=1/R_{HA}(T_H)\times V_A+1/R_{HB}(T_H)\times V_B+1/R_{HC}(T_H)\times V_C+1/R_{HD}(T_H)\times V_D \quad \cdots (32)$$

In view of this, the following Equation (33) shows the inverse number ($1/R_{HI1}$) of the resistance value $R_H$ of the heating element 61 exposed to the mixed gas in a case where the temperature of the heating element 61 is $T_{H1}$. Further, the following Equation (34) shows the inverse number ($1/R_{HI2}$) of the resistance value $R_H$ of the heating element 61 exposed to the mixed gas in a case where the temperature of the heating element 61 is $T_{H2}$. The following Equation (35) shows the inverse number ($1/R_{HI3}$) of the resistance value $R_H$ of the heating element 61 exposed to the mixed gas in a case where the temperature of the heating element 61 is $T_{H3}$.

$$1/R_{HI1}(T_{H1})=1/R_{HA}(T_{H1})\times V_A+1/R_{HB}(T_{H1})\times V_B+1/R_{HC}(T_{H1})\times V_C+1/R_{HD}(T_{H1})\times V_D \quad \cdots (33)$$

$$1/R_{HI2}(T_{H2})=1/R_{HA}(T_{H2})\times V_A+1/R_{HB}(T_{H2})\times V_B+1/R_{HC}(T_{H2})\times V_C+1/R_{HD}(T_{H2})\times V_D \quad \cdots (34)$$

$$1/R_{HI3}(T_{H3})=1/R_{HA}(T_{H3})\times V_A+1/R_{HB}(T_{H3})\times V_B+1/R_{HC}(T_{H3})\times V_C+1/R_{HD}(T_{H3})\times V_D \quad \cdots (35)$$

In Equation (33) to Equation (35), the resistance values $R_{HA}(T_{H1})$, $R_{HB}(T_{H1})$, $R_{HC}(T_{H1})$, $R_{HD}(T_{H1})$, $R_{HA}(T_{H2})$, $R_{HB}(T_{H2})$, $R_{HC}(T_{H2})$, $R_{HD}(T_{H2})$, $R_{HA}(T_{H3})$, $R_{HB}(T_{H3})$, $R_{HC}(T_{H3})$, $R_{HD}(T_{H3})$ of the heating element 61 exposed to the respective gas components may be previously obtained by measurement or the like. In this case, the following Equations (36) to (39) are obtained by solving a simultaneous equation including Equation (10) and Equations (33) to (35). Equations (36) to (39) show the volume fraction $V_A$ of the gas A, the volume fraction $V_B$ of the gas B, the volume fraction $V_C$ of the gas C, and the volume fraction $V_D$ of the gas, respectively. Equations (36) to (39) are functions of the resistance values $R_{HI1}(T_{H1})$, $R_{HI2}(T_{H2})$, $R_{HI3}(T_{H3})$ of the heating element 61 exposed to the mixed gas. In Equations (36) to (39), each of $h_6$, $h_7$, $h_8$, and $h_9$ is indicative of a function.

$$V_A=h_6[1/R_{HI1}(T_{H1}), \quad 1/R_{HI2}(T_{H2}), \quad 1/R_{HI3}(T_{H3})] \quad \cdots (36)$$

$$V_B=h_7[1/R_{HI1}(T_{H1}), \quad 1/R_{HI2}(T_{H2}), \quad 1/R_{HI3}(T_{H3})] \quad \cdots (37)$$

$$V_C=h_8[1/R_{HI1}(T_{H1}), \quad 1/R_{HI2}(T_{H2}), \quad 1/R_{HI3}(T_{H3})] \quad \cdots (38)$$

$$V_D=h_9[1/R_{HI1}(T_{H1}), \quad 1/R_{HI2}(T_{H2}), \quad 1/R_{HI3}(T_{H3})] \quad \cdots (39)$$

Here, Equations (36) to (39) are substituted in Equation (11). As a result, the following Equation (40) is obtained.

$$Q=K_A \times V_A + K_B \times V_B + K_C \times V_C + K_D \times V_D$$
$$=K_A \times h_6[1/R_{HI1}(T_{H1}), \ 1/R_{HI2}(T_{H2}), \ 1/R_{HI3}(T_{H3})]$$
$$+K_B \times h_7[1/R_{HI1}(T_{H1}), \ 1/R_{HI2}(T_{H2}), \ 1/R_{HI3}(T_{H3})]$$
$$+K_C \times h_8[1/R_{HI1}(T_{H1}), \ 1/R_{HI2}(T_{H2}), \ 1/R_{HI3}(T_{H3})]$$
$$+K_D \times h_9[1/R_{HI1}(T_{H1}), \ 1/R_{HI2}(T_{H2}), \ 1/R_{HI3}(T_{H3})] \ ...(40)$$

As shown in Equation (40), the calorific value Q of the mixed gas per unit volume is obtained based on an equation in which the resistance values $R_{HI1}(T_{H1})$, $R_{HI2}(T_{H2})$, $R_{HI3}(T_{H3})$ of the heating element 61 in the case where the temperatures of the heating element 61 are $T_{H1}$, $T_{H2}$, and $T_{H3}$, respectively, are variables. In view of this, the calorific value Q of the mixed gas is obtained based on the following Equation (41). Each of $h_{10}$ and $h_{11}$ is indicative of a function.

$$Q=h_{10}[1/R_{HI1}(T_{H1}), \ 1/R_{HI2}(T_{H2}), \ 1/R_{HI3}(T_{H3})]$$
$$=h_{11}[R_{HI1}(T_{H1}), \ R_{HI2}(T_{H2}), \ R_{HI3}(T_{H3})] \ ...(41)$$

Further, according to Equation (12) and Equations (36) to (39), the calorific value $Q_H$ produced by hydrogen atoms in molecules in the mixed gas, per unit volume, is obtained based on the following Equation (42). $h_{12}$ is indicative of a function.

$$Q_H=h_{12}[R_{HI1}(T_{H1}), \ R_{HI2}(T_{H2}), \ R_{HI3}(T_{H3})] \ ...(42)$$

In view of this, the inventors have found out the following fact. That is, it is possible to easily calculate the calorific value $Q_H$ produced by hydrogen atoms in molecules in the measuring-target mixed gas, per unit volume, by previously obtaining Equation (42) about the mixed gas including the gas A, the gas B, the gas C, and the gas D, even if the volume fraction $V_A$ of the gas A, the volume fraction $V_B$ of the gas B, the volume fraction $V_C$ of the gas C, and the volume fraction $V_D$ of the gas D are unknown. Specifically, the resistance values $R_{HI1}(T_{H1})$, $R_{HI2}(T_{H2})$, $R_{HI3}(T_{H3})$ of the heating element 61 in the case where the heating temperatures of the heating element 61 are $T_{H1}$, $T_{H2}$, and $T_{H3}$, respectively, are measured. The measured $R_{HI1}(T_{H1})$, $R_{HI2}(T_{H2})$, $R_{HI3}(T_{H3})$ are substituted in Equation (42). As a result, it is possible to uniquely obtain the calorific value $Q_H$ produced by hydrogen atoms in molecules in the measuring-target mixed gas, per unit volume. Further, in this case, it is possible to obtain the calorific value $Q_H$ produced by hydrogen atoms in molecules in the mixed gas, per unit volume, only by using the heating element 61, without using the first temperature detector 62 of the microchip 8.

Further, the resistance R is in correlation with the current I. So the calorific value $Q_H$ produced by hydrogen atoms in molecules in the measuring-target mixed gas, per unit volume, is obtained based on the following Equation (43). In Equation (43), $h_{13}$ is indicative of a function. The currents $I_{H1}(T_{H1})$, $I_{H2}(T_{H2})$, $I_{H3}(T_{H3})$ passing through the heating element 61 in the case where the temperatures of the heating element 61 are $T_{H1}$, $T_{H2}$, and $T_{H3}$, respectively, are variables.

$$Q_H=h_{13}[I_{H1}(T_{H1}), \ I_{H2}(T_{H2}), \ I_{H3}(T_{H3})] \ ...(43)$$

Further, the resistance R of the heating element 61 is in correlation with the output signal AD output from the analog-digital converter circuit (hereinafter referred to as "A/D converter circuit") connected to the heating element 61. So the calorific value $Q_H$ produced by hydrogen atoms in molecules in the measuring-target mixed gas, per unit volume, is obtained based on the following Equation (44). In Equation (44), $h_{14}$ is indicative of a function. The output signals $AD_{H1}(T_{H1})$, $AD_{H2}(T_{H2})$, $AD_{H3}(T_{H3})$ output from the A/D converter circuit in the case where the temperatures of the heating element 61 are $T_{H1}$, $T_{H2}$, and $T_{H3}$, respectively, are variables.

$$Q_H=h_{14}[AD_{H1}(T_{H1}), \ AD_{H2}(T_{H2}), \ AD_{H3}(T_{H3})] \ ...(44)$$

As a result, the calorific value $Q_H$ produced by hydrogen atoms in molecules in the measuring-target mixed gas, per unit volume, is based on the following Equation (45). $h_{15}$ is indicative of a function. The electric signals $S_{H1}(T_{H1})$,

$S_{H2}(T_{H2})$, $S_{H3}(T_{H3})$ output from the heating element 61 in the case where the heating temperatures of the heating element 61 are $T_{H1}$, $T_{H2}$, and $T_{H3}$, respectively, are variables.

$$Q_H = h_{15}[S_{H1}(T_{H1}), \ S_{H2}(T_{H2}), \ S_{H3}(T_{H3})] \ \ldots (45)$$

Next, the calorific value $Q_H$ produced by hydrogen atoms in molecules in the mixed gas, per unit volume, is in correlation with the sum of values of the product of the number $N_H$ of hydrogen atoms in each kind of molecules included in the mixed gas, the atomic weight (for example, 1.00794) of hydrogen atoms, and the volume fraction of each kind of molecules in the mixed gas. Because of this, based on Equation (45), the sum $G_H$ of the product of the number $N_H$ of hydrogen atoms in each kind of molecules included in the mixed gas, the atomic weight of hydrogen atoms, and the volume fraction of each kind of molecules is obtained based on the following Equation (46). $h_{16}$ is indicative of a function.

$$G_H = h_{16}[S_{H1}(T_{H1}), \ S_{H2}(T_{H2}), \ S_{H3}(T_{H3})] \ \ldots (46)$$

In view of this, by using Equation (46), it is possible to easily calculate the sum $G_H$ of the product of the number $N_H$ of hydrogen atoms in each kind of molecules included in the measuring-target mixed gas, the atomic weight of hydrogen atoms, and the volume fraction of each kind of molecules, even if the volume fraction $V_A$ of the gas A, the volume fraction $V_B$ of the gas B, the volume fraction $V_C$ of the gas C, and the volume fraction $V_D$ of the gas D are unknown.

Note that the number of the kinds of gas components in the mixed gas may not be limited to four. For example, in a case where the mixed gas includes n kinds of gas components, first, the following Equation (47) is previously obtained. In Equation (47), the electric signals $S_{H1}(T_{H1})$, $S_{H2}(T_{H2})$, $S_{H3}(T_{H3})$, ..., $S_{Hn-1}(T_{Hn-1})$ output from the heating element 61 at at least n-1 kinds of heating temperatures $T_{H1}$, $T_{H2}$, $T_{H3}$, ..., $T_{Hn-1}$, respectively, are variables. Then, the electric signals $S_{H1}(T_{H1})$, $S_{H2}(T_{H2})$, $S_{H3}(T_{H3})$, ..., $S_{Hn-1}(T_{Hn-1})$ output from the heating element 61 at the n-1 kinds of heating temperatures $T_{H1}$, $T_{H2}$, $T_{H3}$, ..., $T_{Hn-1}$ are measured, respectively. Here, the heating element 61 is exposed to the measuring-target mixed gas including n kinds of gas components, the volume fractions of the n kinds of gas components being unknown. The measured $S_{H1}(T_{H1})$, $S_{H2}(T_{H2})$, $S_{H3}(T_{H3})$, ..., $S_{Hn-1}(T_{Hn-1})$ are substituted in Equation (47). As a result, it is possible to uniquely obtain the sum $G_H$ of the product of the number $N_H$ of hydrogen atoms in each kind of molecules included in the measuring-target mixed gas, the atomic weight of hydrogen atoms, and the volume fraction of each kind of molecules. $h_{17}$ is indicative of a function.

$$G_H = h_{17}[S_{H1}(T_{H1}), \ S_{H2}(T_{H2}), \ S_{H3}(T_{H3}), \ \ldots, \ S_{Hn-1}(T_{Hn-1})] \ \ldots (47)$$

For example, mixed gas includes 90 vol% of methane ($CH_4$), 5 vol% of ethane ($C_2H_6$), 1 vol% of propane ($C_3H_8$), 1 vol% of butane ($C_4H_{10}$), 1 vol% of nitrogen ($N_2$), and 2 vol% of carbon dioxide ($CO_2$). In this case, since the number of hydrogen atoms in methane is 4, the product of the number of hydrogen atoms in methane, the atomic weight of hydrogen atoms, and the volume fraction of methane is 3.628584 (=4x1.00794x0.9). Further, since the number of hydrogen atoms in ethane is 6, the product of the number of hydrogen atoms in ethane, the atomic weight of hydrogen atoms, and the volume fraction of ethane is 0.302382 (=6x1.00794x0.05). Further, since the number of hydrogen atoms in propane is 8, the product of the number of hydrogen atoms in propane, the atomic weight of hydrogen atoms, and the volume fraction of propane is 0.0806352 (=8x1.00794x0.01). Further, since the number of hydrogen atoms in butane is 10, the product of the number of hydrogen atoms in butane, the atomic weight of hydrogen atoms, and the volume fraction of butane is 0.100794 (=10x1.00794x0.01). The number of hydrogen atoms in nitrogen is 0. The number of hydrogen atoms in carbon dioxide is 0.

As a result, the sum of: the product of the number of hydrogen atoms in methane, the atomic weight of hydrogen atoms, and the volume fraction of methane; the product of the number of hydrogen atoms in ethane, the atomic weight of hydrogen atoms, and the volume fraction of ethane; the product of the number of hydrogen atoms in propane, the atomic weight of hydrogen atoms, and the volume fraction of propane; and the product of the number of hydrogen atoms in butane, the atomic weight of hydrogen atoms, and the volume fraction of butane is 4.1123952 (=3.628584+0.302382+0.0806352+0.100794).

Note that the mixed gas includes methane ($CH_4$), propane ($C_3H_8$), and in addition alkane ($C_jH_{2j+2}$) other than methane ($CH_4$) and propane ($C_3H_8$) as gas components, where j is a natural number. In this case, even if the alkane ($C_jH_{2j+2}$) other than methane ($CH_4$) and propane ($C_3H_8$) is considered as a mixture of methane ($CH_4$) and propane ($C_3H_8$), it does not affect calculation of Equation (47). For example, as shown in the following Equations (48) to (51), ethane ($C_2H_6$), butane ($C_4H_{10}$), pentane ($C_5H_{12}$), or hexane ($C_6H_{14}$) may be considered as a mixture of methane ($CH_4$)

and propane ($C_3H_8$), the mixture being multiplied by a predetermined coefficient, and Equation (47) may be calculated.

$$C_2H_6=0.5CH_4+0.5C_3H_8 \quad \ldots (48)$$

$$C_4H_{10}=-0.5CH_4+1.5C_3H_8 \quad \ldots (49)$$

$$C_5H_{12}=-1.0CH_4+2.0C_3H_8 \quad \ldots (50)$$

$$C_6H_{14}=-1.5CH_4+2.5C_3H_8 \quad \ldots (51)$$

The mixed gas, which includes n kinds of gas components, includes methane ($CH_4$), propane ($C_3H_8$), and in addition z kinds of alkane ($C_jH_{2j+2}$) other than methane ($CH_4$) and propane ($C_3H_8$) as the gas components, where z is a natural number. In this case, an equation, in which electric signals $S_H$ output from the heating element 61 at at least n-z-1 kinds of heating temperatures are variables, may thus be obtained.

As a matter of course, it is possible to use Equation (47) in a case where the kinds of gas components in the mixed gas used for calculation of Equation (47) are the same as the kinds of gas components in the measuring-target mixed gas. Further, it is also possible to use Equation (47) in a case where the number of kinds of gas components in the measuring-target mixed gas is less than n, and where the number of gas components in the mixed gas used for calculation of Equation (47) is less than n. For example, the mixed gas used for calculation of Equation (47) includes four kinds of gas components, i.e., methane ($CH_4$), propane ($C_3H_8$), nitrogen ($N_2$), and carbon dioxide ($CO_2$). In this case, it is also possible to use Equation (47) in a case where the measuring-target mixed gas does not include nitrogen ($N_2$), but only includes three kinds of gas components, i.e., methane ($CH_4$), propane ($C_3H_8$), and carbon dioxide ($CO_2$).

Further, the mixed gas used for calculation of Equation (47) includes methane ($CH_4$) and propane ($C_3H_8$) as gas components. In this case, it is possible to use Equation (47) in a case where the measuring-target mixed gas includes alkane ($C_jH_{2j+2}$), which is not included in the mixed gas used for calculation of Equation (47). The reason is as follows. That is, as described above, alkane ($C_jH_{2j+2}$) other than methane ($CH_4$) and propane ($C_3H_8$) may be considered as a mixture of methane ($CH_4$) and propane ($C_3H_8$).

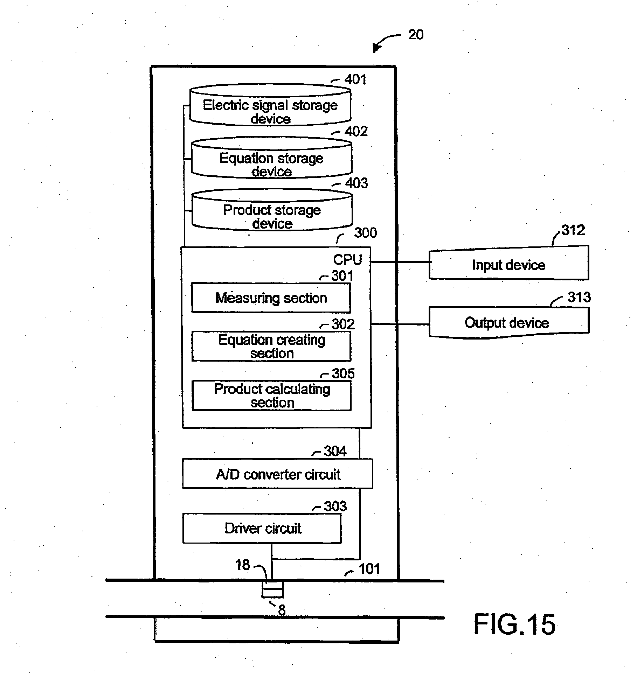

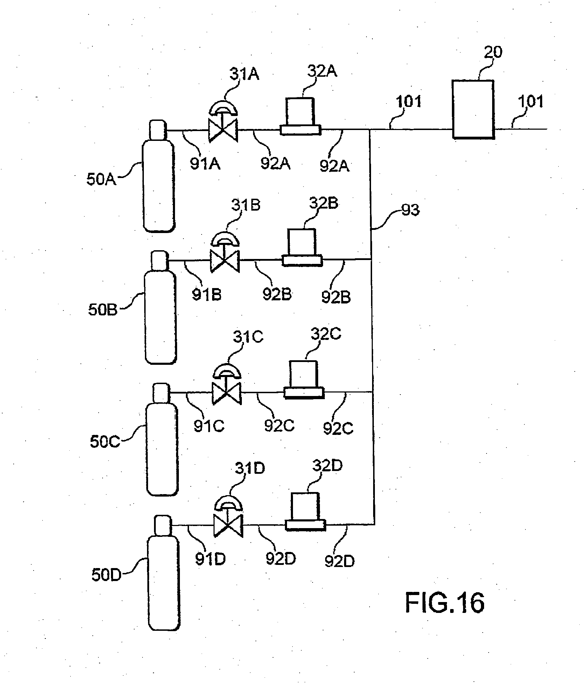

Here, each of Fig. 15 and Fig. 16 shows a gas measuring system 20 according to the first embodiment. The gas measuring system 20 includes a pipe 101 and the microchip 8. Each of a plurality of kinds of sample mixed gas flows in the pipe 101. The microchip 8 is arranged in the pipe 101. The microchip 8 includes the heating element 61 of Fig. 1. The heating element 61 produces heat at a plurality of heating temperatures $T_H$. Note that each sample mixed gas includes a plurality of kinds of gas components. Further, the gas measuring system 20 of Fig. 15 includes a measuring section 301. The measuring section 301 measures values of the electric signals $S_H$ output from the heating element 61 at a plurality of heating temperatures $T_H$, respectively. Further, the gas measuring system 20 includes an equation creating section 302. The equation creating section 302 creates an equation based on a known value of the sum of the product of the number $N_H$ of hydrogen atoms in each kind of molecules included in the mixed gas including a plurality of kinds of gas, the atomic weight of hydrogen atoms, and the volume fraction of each kind of molecules, and based on values of the electric signals output from the heating element 61 at a plurality of heating temperatures, respectively. The equation created by the equation creating section 302 includes the electric signals $S_H$ output from the heating element 61 at a plurality of heating temperatures $T_H$, respectively, as independent variables. The equation created by the equation creating section 302 further includes the sum of the product of the number $N_H$ of hydrogen atoms in each kind of molecules included in the gas, the atomic weight of hydrogen atoms, and the volume fraction of each kind of molecules, as a dependent variable.

Four kinds of sample mixed gas are used. The sum of the product of the number $N_H$ of hydrogen atoms in each kind of molecules included in each gas, the atomic weight of hydrogen atoms, and the volume fraction of each kind of molecules of one kind of sample mixed gas is different from those of any other kind of sample mixed gas. In this case, as shown in Fig. 16, a first gas cylinder 50A, a second gas cylinder 50B, a third gas cylinder 50C, and a fourth gas cylinder 50D are prepared. The first gas cylinder 50A stores a first sample mixed gas. The second gas cylinder 50B stores a second sample mixed gas. The third gas cylinder 50C stores a third sample mixed gas. The fourth gas cylinder 50D stores a fourth sample mixed gas. A first gas-pressure regulator 31A is connected to the first gas cylinder 50A via a pipe 91A. The first gas-pressure regulator 31A is capable of obtaining a first sample mixed gas from the first gas cylinder 50A, which is regulated at low pressure (e.g., 0.2 MPa). Further, a first flow controller 32A is connected to the first gas-pressure regulator 31A via a pipe 92A. The first flow controller 32A controls a flow rate of the first sample mixed gas, which is sent to the gas measuring system 20 via the pipe 92A and the pipe 101.

A second gas-pressure regulator 31B is connected to the second gas cylinder 50B via a pipe 91B. Further, a second flow controller 32B is connected to the second gas-pressure regulator 31B via a pipe 92B. The second flow controller 32B controls a flow rate of the second sample mixed gas, which is sent to the gas measuring system 20 via the pipes 92B, 93, 101.

A third gas-pressure regulator 31C is connected to the third gas cylinder 50C via a pipe 91C. Further, a third flow controller 32C is connected to the third gas-pressure regulator 31C via the pipe 92C. The third flow controller 32C controls a flow rate of the third sample mixed gas, which is sent to the gas measuring system 20 via the pipes 92C, 93, 101.

A fourth gas-pressure regulator 31D is connected to the fourth gas cylinder 50D via a pipe 91D. Further, a fourth flow controller 32D is connected to the fourth gas-pressure regulator 31D via a pipe 92D. The fourth flow controller 32D controls a flow rate of the fourth sample mixed gas, which is sent to the gas measuring system 20 via the pipe 92D, 93, 101.

Each of the first sample mixed gas to the fourth sample mixed gas is, for example, natural gas or city gas. Each of the first sample mixed gas to the fourth sample mixed gas includes four kinds of gas components (for example, methane ($CH_4$), propane ($C_3H_8$), nitrogen ($N_2$), and carbon dioxide ($CO_2$)) at different volume fractions.

The first sample mixed gas is supplied to the pipe 101 of Fig. 15. In this situation, a driver circuit 303 of Fig. 15 sequentially provides drive powers $P_{H1}$, $P_{H2}$, $P_{H3}$ to the heating element 61 of the microchip 8 of Fig. 1 and Fig. 2. In a case where the drive powers $P_{H1}$, $P_{H2}$, $P_{H3}$ are supplied to the heating element 61, the heating element 61 in the first sample mixed gas sequentially produces heat at a temperature $T_{H1}$ (100°C), a temperature $T_{H2}$ (150°C), and a temperature $T_{H3}$ (200°C), for example. The heating element 61 outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$.

The first sample mixed gas is removed from the pipe 101. After that, the second sample mixed gas to the fourth sample mixed gas are sequentially supplied to the pipe 101. In a case where the second sample mixed gas is supplied to the pipe 101, the heating element 61 of the microchip 8 of Fig. 1 and Fig. 2 outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$. The third sample mixed gas is supplied to the pipe 101 of Fig. 15. In this case, the heating element 61 of the microchip 8 of Fig. 1 and Fig.

2 outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$. The fourth sample mixed gas is supplied to the pipe 101 of Fig. 15. In this case, the heating element 61 of the microchip 8 of Fig. 1 and Fig. 2 outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$.

Note that each sample mixed gas includes n kinds of gas components. In this case, the heating element 61 of the microchip 8 of Fig. 1 and Fig. 2 produces heat at at least n-1 kinds of different temperatures. Note that, as described above, alkane ($C_jH_{2j+2}$) other than methane ($CH_4$) and propane ($C_3H_8$) may be considered as a mixture of methane ($CH_4$) and propane ($C_3H_8$). The sample mixed gas, which includes n kinds of gas components, includes methane ($CH_4$), propane ($C_3H_8$), and in addition z kinds of alkane ($C_jH_{2j+2}$) as gas components, where z is a natural number. In this case, the heating element 61 thus produces heat at at least n-z-1 kinds of different temperatures.

As shown in Fig. 15, the microchip 8 is connected to a central processing unit (CPU) 300. The CPU 300 includes the measuring section 301. An electric signal storage device 401 is connected to the CPU 300. The measuring section 301 measures values of the electric signal $S_{H1}(T_{H1})$ at the heating temperature $T_{H1}$, the electric signal $S_{H2}(T_{H2})$ at the heating temperature $T_{H2}$, and the electric signal $S_{H3}(T_{H3})$ at the heating temperature $T_{H3}$, which are output from the heating element 61. The measuring section 301 stores the measured values in the electric signal storage device 401.

Note that the electric signal $S_H$ output from the heating element 61 is any one of the resistance value $R_H$ of the heating element 61, the current $I_H$ passing through the heating element 61, and an output signal $AD_H$ output from an A/D converter circuit 304 connected to the heating element 61.

The equation creating section 302 of the CPU 300 collects: the known value of the sum of the product of the number $N_H$ of hydrogen atoms in each kind of molecules included in the first sample mixed gas, the atomic weight of hydrogen atoms, and the volume fraction of each kind of molecules; the known value of the sum of the product of the number $N_H$ of hydrogen atoms in each kind of molecules included in the second sample mixed gas, the atomic weight of hydrogen atoms, and the volume fraction of each kind of molecules; the known value of the sum of the product of the number $N_H$ of hydrogen atoms in each kind of molecules included in the third sample mixed gas, the atomic weight of hydrogen atoms, and the volume fraction of each kind of molecules; the known value of the sum of the product of the number $N_H$ of hydrogen atoms in each kind of molecules included in the fourth sample mixed gas, the atomic weight of hydrogen atoms, and the volume fraction of each kind of molecules; and the plurality of measured values of the electric signals $S_{H1}(T_{H1})$, $S_{H2}(T_{H2})$, $S_{H3}(T_{H3})$ output from the heating element 61, for example. Further, the equation creating section 302 calculates an equation based on the collected values of the sum of the product and based on the collected values of the electric signals $S_H$ by means of multivariate statistics. The equation created by the equation creating section 302 includes the electric signals $S_{H1}(T_{H1})$, $S_{H2}(T_{H2})$, $S_{H3}(T_{H3})$ output from the heating element 61, as independent variables. The equation further includes the sum of the product of the number $N_H$ of hydrogen atoms in each kind of molecules included in the gas, the atomic weight of hydrogen atoms, and the volume fraction of each kind of molecules, as a dependent variable.

Note that, examples of "multivariate statistics" include support vector regression and multiple regression analysis disclosed in "A Tutorial on Support Vector Regression" (NeuroCOLT Technical Report (NC-TR-98-030), 1998) by A. J Smola and B. Scholkopf and fuzzy quantification theory of second kind disclosed in Japanese Patent Application Laid-open No. H05-141999.

The gas measuring system 20 further includes an equation storage device 402 connected to the CPU 300. The equation storage device 402 stores the equation created by the equation creating section 302. Further, an input device 312 and an output device 313 are connected to the CPU 300. Examples of the input device 312 include a keyboard and a pointing device such as a mouse. Examples of the output device 313 include a printer and an image display device such as a liquid crystal display and a monitor.

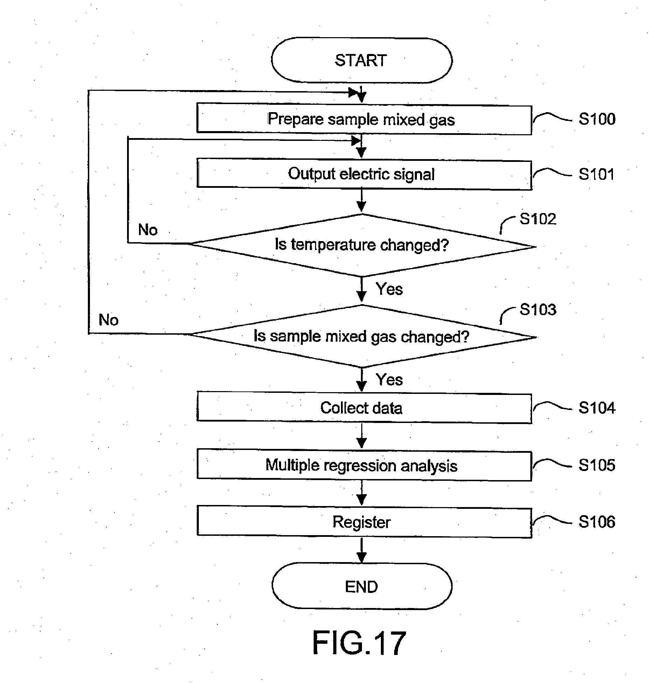

Next, with reference to a flowchart of Fig. 17, a method of creating an equation according to the first embodiment will be described. The equation is used to calculate the sum of the product of the number $N_H$ of hydrogen atoms in each kind of molecules included in each gas, the atomic weight of hydrogen atoms, and the volume fraction of each kind of molecules.

(a) In Step S100, valves of the second to fourth flow controllers 32B to 32D of Fig. 16 are closed, and a valve of the first flow controller 32A is open. The first sample mixed gas is introduced in the pipe 101 of Fig. 15. In Step S101, the driver circuit 303 supplies drive power $P_{H1}$ to the heating element 61 of Fig. 1 and Fig. 2. As a result, the heating element 61 produces heat at 100°C. The measuring section 301 of Fig. 15 receives an electric signal $S_{H1}(T_{H1})$ output from the heating element 61, which produces heat at 100°C, and stores the value of the electric signal $S_{H1}(T_{H1})$ in the electric signal storage device 401.

(b) In Step S102, the driver circuit 303 determines whether the temperature of the heating element 61 of Fig. 1 and Fig. 2 is changed. If the temperature of the heating element 61 is not changed to 150°C and 200°C, Step S101 is performed again. The driver circuit 303 of Fig. 15 causes the heating element 61 of Fig. 1 and Fig. 2 to produce heat at 150°C. The measuring section 301 of Fig. 15 receives an electric signal $S_{H2}(T_{H2})$ output from the heating element 61, which is exposed to the first sample mixed gas and produces heat at 150°C, and stores the value of the electric signal $S_{H2}(T_{H2})$ in the electric signal storage device 401.

(c) In Step S102, the driver circuit 303 determines whether the temperature of the heating element 61 of Fig. 1 and Fig. 2 is changed again. If the temperature of the heating element 61 is not changed to 200°C, Step S101 is performed again. The driver circuit 303 of Fig. 15 causes the heating element 61 of Fig. 1 and Fig. 2 to produce heat at 200°C. The measuring section 301 of Fig. 15 receives an electric signal $S_{H3}(T_{H3})$ output from the heating element 61, which is exposed to the first sample mixed gas and produces heat at 200°C, and stores the value of the electric signal $S_{H3}(T_{H3})$ in the electric signal storage device 401.

(d) If the temperature of the heating element 61 is changed, Step S102 is finished and Step S103 is performed. In Step S103, whether the sample mixed gas is changed is determined. If the sample mixed gas is not changed to the second sample mixed gas to the fourth sample mixed gas, Step S100 is performed again. In Step S100, the first flow controller 32A of Fig. 16 is closed, the valves of the third to fourth flow controllers 32C to 32D are closed, and the valve of the second flow controller 32B is open. The second sample mixed gas is introduced in the pipe 101 of Fig. 15.

(e) Similar to the case of the first sample mixed gas, the loop of Step S101 to Step S102 is repeated. The heating element 61 is exposed to the second sample mixed gas, and produces heat at 100°C, 150°C, and 200°C. The measuring section 301 receives the electric signals $S_{H1}(T_{H1})$, $S_{H2}(T_{H2})$, $S_{H3}(T_{H3})$ output from the heating element 61. The measuring section 301 stores the values of the electric signals $S_{H1}(T_{H1})$, $S_{H2}(T_{H2})$, $S_{H3}(T_{H3})$ in the electric signal storage device 401.

(f) After that, the loop of Step S100 to Step S103 is repeated. The heating element 61 is exposed to the third sample mixed gas supplied to the pipe 101. The heating element 61 produces heat at 100°C, 150°C, and 200°C. Values of the electric signals $S_{H1}(T_{H1})$, $S_{H2}(T_{H2})$, $S_{H3}(T_{H3})$ output from the heating element 61 are thus stored in the electric signal storage device 401. Further, the heating element 61 is exposed to the fourth sample mixed gas supplied to the pipe 101. The heating element 61 produces heat at 100°C, 150°C, and 200°C. Values of the electric signals $S_{H1}(T_{H1})$, $S_{H2}(T_{H2})$, $S_{H3}(T_{H3})$ output from the heating element 61 are thus stored in the electric signal storage device 401.

(g) In Step S104, the input device 312 inputs, in the equation creating section 302: the known value of the sum of the product of the number $N_H$ of hydrogen atoms in each kind of molecules included in the first sample mixed gas, the atomic weight of hydrogen atoms, and the volume fraction of each kind of molecules; the known value of the sum of the product of the number $N_H$ of hydrogen atoms in each kind of molecules included in the second sample mixed gas, the atomic weight of hydrogen atoms, and the volume fraction of each kind of molecules; the known value of the sum of the product of the number $N_H$ of hydrogen atoms in each kind of molecules included in the third sample mixed gas, the atomic weight of hydrogen atoms, and the volume fraction of each kind of molecules; and the known value of the sum of the product of the number $N_H$ of hydrogen atoms in each kind of molecules included in the fourth sample mixed gas, the atomic weight of hydrogen atoms, and the volume fraction of each kind of molecules. Further, the equation creating section 302 retrieves the plurality of measured values of the electric signals $S_{H1}(T_{H1})$, $S_{H2}(T_{H2})$, $S_{H3}(T_{H3})$ output from the heating element 61, from the electric signal storage device 401.

(h) In Step S105, the equation creating section 302 performs multiple regression analysis based on the known values of the sum of the product, and based on the plurality of measured values of the electric signals $S_{H1}(T_{H1})$, $S_{H2}(T_{H2})$, $S_{H3}(T_{H3})$ output from the heating element 61. As the result of multiple regression analysis, the equation creating section 302 calculates an equation including the electric signals $S_{H1}(T_{H1})$, $S_{H2}(T_{H2})$, $S_{H3}(T_{H3})$ output from the heating element 61, as independent variables, and the sum of the product of the number $N_H$ of hydrogen atoms in each kind of molecules included in the gas, the atomic weight of hydrogen atoms, and the volume fraction of each kind of molecules, as a dependent variable. After that, in Step S106, the equation creating section 302 stores the created equation in the equation storage device 402. The method of creating an equation according to the first embodiment is thus finished.

As described above, according to the first embodiment of the present invention, it is possible to create an equation capable of uniquely calculating the sum of the product of the number $N_H$ of hydrogen atoms in each kind of molecules included in the measuring-target mixed gas, the atomic weight of hydrogen atoms, and the volume fraction of each kind of molecules.

Next, functions of the gas measuring system 20 of Fig. 15 according to the first embodiment in the case of measuring the sum of the product of the number $N_H$ of hydrogen atoms in each kind of molecules included in the measuring-target mixed gas, the atomic weight of hydrogen atoms, and the volume fraction of each kind of molecules, will be described. The measuring-target mixed gas being natural gas or city gas is introduced in the pipe 101. For example, the measuring-target mixed gas includes methane ($CH_4$), propane ($C_3H_8$), nitrogen ($N_2$), carbon dioxide ($CO_2$), and the like of unknown volume fractions. Next, the driver circuit 303 of Fig. 15 sequentially supplies the drive powers $P_{H1}$, $P_{H2}$, $P_{H3}$ to the heating element 61. In the case where the drive powers $P_{H1}$, $P_{H2}$, $P_{H3}$ are supplied to the heating element 61, the heating element 61 exposed to the measuring-target mixed gas sequentially produces heat at the temperature $T_{H1}$ (100°C), the temperature $T_{H2}$ (150°C), and the temperature $T_{H3}$ (200°C), for example. The heating element 61 outputs an electric signal $S_{H1}(T_{H1})$ at the heating temperature $T_{H1}$, an electric signal $S_{H2}(T_{H2})$ at the heating temperature $T_{H2}$, and an electric signal $S_{H3}(T_{H3})$ at the heating temperature $T_{H3}$.

The measuring section 301 of Fig. 15 measures values of the electric signal $S_{H1}(T_{H1})$ at the heating temperature $T_{H1}$, the electric signal $S_{H2}(T_{H2})$ at the heating temperature $T_{H2}$, and the electric signal $S_{H3}(T_{H3})$ at the heating temperature $T_{H3}$, which are output from the heating element 61 exposed to the measuring-target mixed gas supplied to the pipe 101. The measuring section 301 stores the measured values in the electric signal storage device 401.

As described above, the equation storage device 402 stores an equation including the electric signal $S_{H1}(T_{H1})$ output from the heating element 61 at the heating temperature $T_{H1}$ (100°C), the electric signal $S_{H2}(T_{H2})$ output from the heating element 61 at the heating temperature $T_{H2}$ (150°C), and the electric signal $S_{H3}(T_{H3})$ output from the heating element 61 at the heating temperature $T_{H3}$ (200°C), as independent variables, and further including the sum of the product of the number $N_H$ of hydrogen atoms in each kind of molecules included in the gas, the atomic weight of hydrogen atoms, and the volume fraction of each kind of molecules, as a dependent variable.

The gas measuring system 20 according to the first embodiment further includes a product calculating section 305. The product calculating section 305 substitutes the measured values of the electric signals $S_{H1}(T_{H1})$, $S_{H2}(T_{H2})$, $S_{H3}(T_{H3})$ output from the heating element 61 in the independent variables in the equation stored in the equation storage device 402. The independent variables indicate the electric signals $S_{H1}(T_{H1})$, $S_{H2}(T_{H2})$, $S_{H3}(T_{H3})$ output from the heating element 61. The product calculating section 305 calculates the sum of the product of the number $N_H$ of hydrogen atoms in each kind of molecules included in the measuring-target mixed gas, the atomic weight of hydrogen atoms, and the volume fraction of each kind of molecules. A product storage device 403 is further connected to the CPU 300. The product storage device 403 stores the sum of the product calculated by the product calculating section 305.

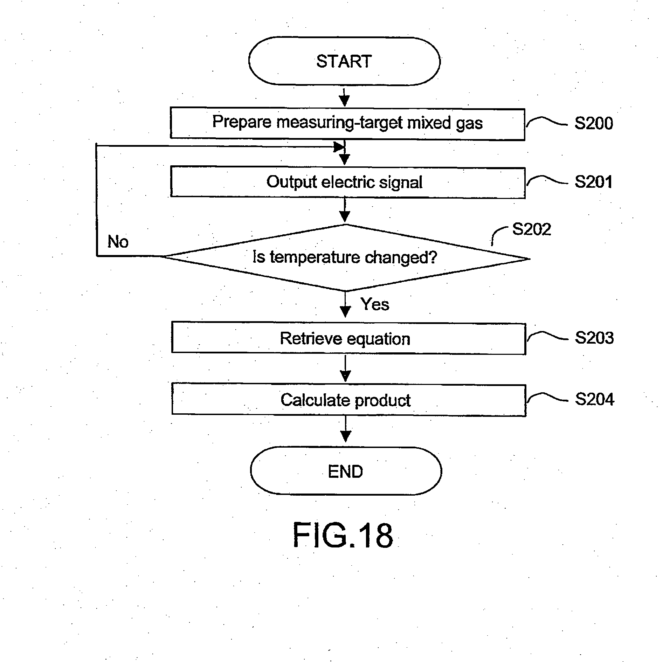

Next, with reference to a flowchart of Fig. 18, a gas measuring method according to the first embodiment will be described.

(a) In Step S200, the measuring-target mixed gas is introduced in the pipe 101 of Fig. 15. In Step S201, the driver circuit 303 supplies drive power $P_{H1}$ to the heating element 61 of Fig. 1 and Fig. 2. As a result, the heating element 61 produces heat at 100°C. The measuring section 301 of Fig. 15 receives an electric signal $S_{H1}(T_{H1})$ from the heating element 61 exposed to the measuring-target mixed gas, which produces heat at 100°C. The measuring section 301 stores the value of the electric signal $S_{H1}(T_{H1})$ in the electric signal storage device 401.

(b) In Step S202, the driver circuit 303 of Fig. 15 determines whether the temperature of the heating element 61 of Fig. 1 and Fig. 2 is changed. If the temperature of the heating element 61 is not changed to 150°C and 200°C, Step S201 is performed again. The driver circuit 303 supplies drive power $P_{H2}$ to the heating element 61 of Fig. 1 and Fig. 2 to thereby cause the heating element 61 of Fig. 1 and Fig. 2 to produce heat at 150°C. The measuring section 301 of Fig. 15 receives an electric signal $S_{H2}(T_{H2})$ output from the heating element 61 exposed to the measuring-target mixed gas, which produces heat at 150°C, and stores the value of electric signal $S_{H2}(T_{H2})$ in the electric signal storage device 401.

(c) In Step S202, the driver circuit 303 determines whether the temperature of the heating element 61 of Fig. 1 and Fig. 2 is changed again. If the temperature of the heating element 61 is not changed to 200°C, Step S201 is performed again. The driver circuit 303 supplies drive power $P_{H3}$ to the heating element 61 of Fig. 1 and Fig. 2 to thereby cause the heating element 61 of Fig. 1 and Fig. 2 to produce heat at 200°C. The measuring section 301 of Fig. 15 receives an electric signal $S_{H3}(T_{H3})$ output from the heating element 61 exposed to the measuring-target mixed gas, which produces heat at 200°C, and stores the value of the electric signal $S_{H3}(T_{H3})$ in the electric signal storage device 401.

(d) If the temperature of the heating element 61 is changed, Step S202 is finished and Step S203 is performed. In Step S203, the product calculating section 305 of Fig. 15 retrieves, from the equation storage device 402, the equation including the electric signals $S_{H1}(T_{H1})$, $S_{H2}(T_{H2})$, $S_{H3}(T_{H3})$ output from the heating element 61, as independent variables, and the sum of the product of the number $N_H$ of hydrogen atoms in each kind of molecules included in the gas, the atomic weight of hydrogen atoms, and the volume fraction of each kind of molecules, as a dependent variable. Further, the product calculating section 305 retrieves, from the electric signal storage device 401, the measured values of the electric signals $S_{H1}(T_{H1})$, $S_{H2}(T_{H2})$, $S_{H3}(T_{H3})$ output from the heating element 61 exposed to the measuring-target mixed gas.

(e) In Step S204, the product calculating section 305 substitutes measured values in the independent variables in the equation. The independent variables indicate the electric signals $S_{H1}(T_{H1})$, $S_{H2}(T_{H2})$, $S_{H3}(T_{H3})$. The product calculating section 305 calculates the sum of the product of the number $N_H$ of hydrogen atoms in each kind of molecules included in the measuring-target mixed gas, the atomic weight of hydrogen atoms, and the volume fraction of each kind of molecules. After that, the product calculating section 305 stores the sum of the product thus calculated in the product storage device 403. The gas measuring method according to the first embodiment is thus finished.

As described above, according to the first embodiment of the present invention, it is possible to measure the sum of the product of the number $N_H$ of hydrogen atoms in each kind of molecules included in the measuring-target mixed gas, the atomic weight of hydrogen atoms, and the volume fraction of each kind of molecules, based on the values of the electric signals $S_{H1}(T_{H1})$, $S_{H2}(T_{H2})$, $S_{H3}(T_{H3})$ output from the heating element 61 exposed to the measuring-target mixed gas.

(First example of first embodiment)

First, 40 kinds of sample mixed gas were prepared. Compositions of the 40 kinds of prepared sample mixed gas were known. Each of the 40 kinds of sample mixed gas included, as gas components, at least one of or all of methane, ethane, propane, butane, pentane, hexane, nitrogen, and carbon dioxide. Next, the 40 kinds of sample mixed gas were used, and a plurality of measured values of an electric signal $S_I$ from the first temperature detector 62 of Fig. 1, and a plurality of measured values of the electric signals $S_{H1}(T_{H1})$, $S_{H2}(T_{H2})$, $S_{H3}(T_{H3})$, $S_{H4}(T_{H4})$, $S_{H5}(T_{H5})$ output from the heating element 61 were obtained.

After that, an equation was created by means of support vector regression based on the known value of the sum of the product of the number of hydrogen atoms in each kind of molecules included in each of the 40 kinds of sample mixed gas, the atomic weight of hydrogen atoms, and the volume fraction of each kind of molecules, based on the plurality of measured values of the electric signal $S_I$ output from the first temperature detector 62, and based on the plurality of measured values of the electric signals $S_{H1}(T_{H1})$, $S_{H2}(T_{H2})$, $S_{H3}(T_{H3})$, $S_{H4}(T_{H4})$, $S_{H5}(T_{H5})$ output from the heating element 61. The electric signal $S_I$ output from the first temperature detector 62, and the electric signals $S_{H1}(T_{H1})$, $S_{H2}(T_{H2})$, $S_{H3}(T_{H3})$, $S_{H4}(T_{H4})$, $S_{H5}(T_{H5})$ output from the heating element 61 were independent variables. The sum of the product of the number of hydrogen atoms in each kind of molecules included in the gas, the atomic weight of hydrogen atoms, and the volume fraction of each kind of molecules was a dependent variable.

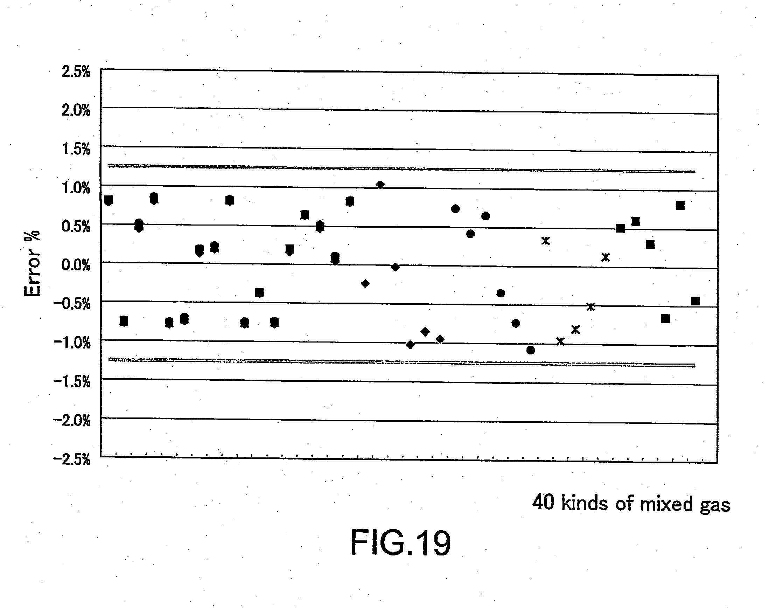

In creating the equation, the number of calibration points may be arbitrarily determined (e.g., 3 to 5). The sum of the product of the number of hydrogen atoms in each of the 40 kinds of sample mixed gas, the atomic weight of hydrogen atoms, and the volume fraction of each kind of molecules was calculated by using the created equation. The sum was compared with the true value. As shown in Fig. 19, an error was plus/minus 1.25%.

(Second example of first embodiment)

Similar to the first example, 40 kinds of sample mixed gas were prepared. Compositions of the 40 kinds of prepared sample mixed gas were known. Next, the 40 kinds of sample mixed gas were used, and a plurality of measured values of an electric signal $S_I$ from the first temperature detector 62 of Fig. 1, and a plurality of measured values of the electric signals $S_{H1}(T_{H1})$, $S_{H2}(T_{H2})$, $S_{H3}(T_{H3})$, $S_{H4}(T_{H4})$, $S_{H5}(T_{H5})$ output from the heating element 61 were obtained.

After that, as shown in Equation (48) and Equation (49), each of ethane and butane was considered as a mixture of methane and propane. The sum of the product of the number of hydrogen atoms in each kind of molecules included in each of the 40 kinds of sample mixed gas, the atomic weight of hydrogen atoms, and the volume fraction of each kind of molecules was calculated by using the created equation. Compositions of the each of the 40 kinds of sample mixed gas were known. For example, one kind of sample mixed gas included 90 vol% of methane, 5 vol% of ethane, 1 vol% of propane, 1 vol% of butane, 1 vol% of nitrogen, and 2% of carbon dioxide. In this case, ethane decomposed into methane and propane. Butane decomposed into methane and propane. Then, the volume of methane was considered as 92 vol%, which was the sum of 90 vol% of original methane, 0.5x5 vol% of methane decomposed from ethane, and -0.5x1 vol% of methane decomposed from butane. Further, the volume of propane was considered as 5 vol%, which was the sum of 0.5x5 vol% of propane decomposed from ethane, 1 vol% of original propane, and 1.5x1 vol% of propane decomposed from butane.

Further, an equation was created by means of support vector regression based on the calculated sum of the products, based on the plurality of measured values of the electric signal $S_I$ output from the first temperature detector 62, and based on the plurality of measured values of the electric signals $S_{H1}(T_{H1})$, $S_{H2}(T_{H2})$, $S_{H3}(T_{H3})$, $S_{H4}(T_{H4})$, $S_{H5}(T_{H5})$ output from the heating element 61. In the created equation, the electric signal $S_I$ output from the first temperature detector 62, and the electric signals $S_{H1}(T_{H1})$, $S_{H2}(T_{H2})$, $S_{H3}(T_{H3})$, $S_{H4}(T_{H4})$, $S_{H5}(T_{H5})$ output from the heating element 61 were the independent variables. The sum of the product of the number of hydrogen atoms in each kind of molecules included in the gas, the atomic weight of hydrogen atoms, and the volume fraction of each kind of molecules was a dependent variable.

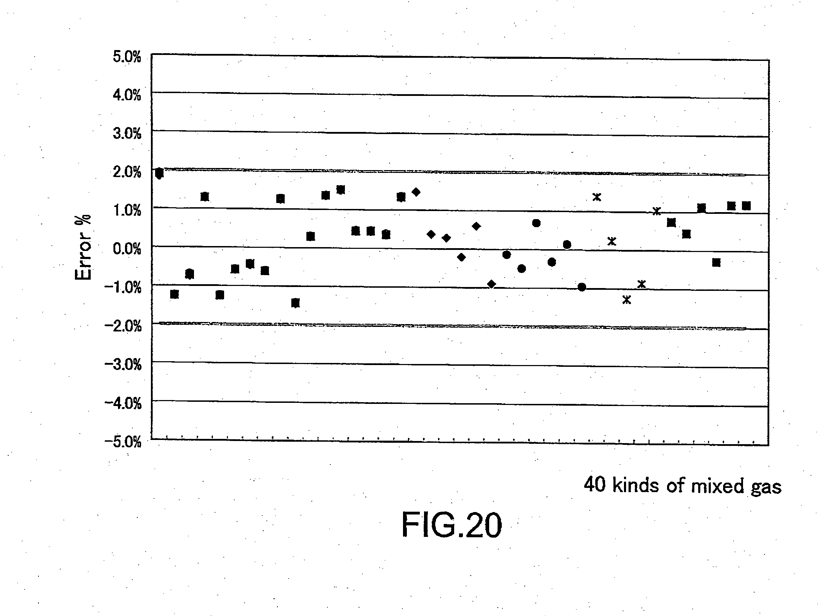

In creating the equation, the number of calibration points may be arbitrarily determined (e.g., 3 to 5). The sum of the product of the number of hydrogen atoms in each kind of molecules included in each of the 40 kinds of sample mixed gas, the atomic weight of hydrogen atoms, and the volume fraction of each kind of molecules was calculated by using the created equation. The sum was compared with the true value. As shown in Fig. 20, an error was plus/minus 2.0%.

(Second Embodiment)

The atmosphere gas around the microchip 8 of Fig. 1 to Fig. 4 is mixed gas including four kinds of gas components, i.e., the gas A, the gas B, the gas C, and the gas D. $K_{AC}$ is indicative of a calorific value per unit volume of heat produced by carbon atoms in the gas A. $K_{BC}$ is indicative of a calorific value per unit volume of heat produced by carbon atoms in the gas B. $K_{CC}$ is indicative of a calorific value per unit volume of heat produced by carbon atoms in the gas C. $K_{DC}$ is indicative of a calorific value per unit volume of heat produced by carbon atoms in the gas D. In this case, a calorific value $Q_C$ per unit volume of heat produced by carbon atoms in the mixed gas equals to the sum of values, which are obtained by multiplying the volume fractions of the gas components by the calorific values of heat produced by carbon atoms in the gas components, per unit volume. In view of this, the following Equation (52) shows the calorific value $Q_C$ per unit volume of heat produced by carbon atoms in molecules in the mixed gas.

$$Q_C = K_{AC} \times V_A + K_{BC} \times V_B + K_{CC} \times V_C + K_{DC} \times V_D \quad \ldots (52)$$

In view of Equation (52) and Equations (19) to (22), the following Equation (53) shows the calorific value $Q_C$ per unit volume of heat produced by carbon atoms in molecules in the mixed gas. $g_3$ is indicative of a function.

$$Q_C = g_3[M_{I1}(T_{H1}), \; M_{I2}(T_{H2}), \; M_{I3}(T_{H3})] \quad \ldots (53)$$

Further, as described above, the heat-radiation coefficient $M_I$ of the mixed gas depends on the electric signals $S_{H1}(T_{H1})$, $S_{H2}(T_{H2})$, $S_{H3}(T_{H3})$ output from the heating element 61 in the case where the heating temperatures of the heating element 61 are $T_{H1}$, $T_{H2}$, and $T_{H3}$, respectively, and depends on the electric signal $S_I$ output from the first temperature detector 62 exposed to the mixed gas. In view of this, the following Equation (54) also shows the calorific value $Q_C$ per unit volume of heat produced by carbon atoms in molecules in the mixed gas. $h_{18}$ is indicative of a function.

$$Q_C = h_{18}[S_{H1}(T_{H1}), \ S_{H2}(T_{H2}), \ S_{H3}(T_{H3}), \ S_I] \ \ldots (54)$$

Further, as shown in the following Equation (55), the independent variable of the electric signal $S_I$ output from the first temperature detector 62 may be omitted. $h_{19}$ is indicative of a function.

$$Q_C = h_{19}[S_{H1}(T_{H1}), \ S_{H2}(T_{H2}), \ S_{H3}(T_{H3})] \ \ldots (55)$$

Further, the calorific value $Q_C$ per unit volume of heat produced by carbon atoms in molecules in the mixed gas is in correlation with the sum of the product of the number $N_C$ of carbon atoms in each kind of molecules included in the mixed gas, the atomic weight of carbon atoms (for example, 12.0107), and the volume fraction of each kind of molecules. In view of this, the following Equation (56) shows the sum $G_C$ of the product of the number $N_C$ of carbon atoms in each kind of molecules included in the mixed gas, the atomic weight of carbon atoms, and the volume fraction of each kind of molecules, based on Equation (55). $h_{20}$ is indicative of a function.

$$G_C = h_{20}[S_{H1}(T_{H1}), \ S_{H2}(T_{H2}), \ S_{H3}(T_{H3})] \ \ldots (56)$$

Further, the following Equation (57) shows the sum $G_C$ of the product of the number $N_C$ of carbon atoms in each kind of molecules included in the mixed gas, which includes n kinds of gas components, the atomic weight of carbon atoms, and the volume fraction of each kind of molecules. $h_{21}$ is indicative of a function.

$$G_C = h_{21}[S_{H1}(T_{H1}), \ S_{H2}(T_{H2}), \ S_{H3}(T_{H3}), \ \ldots, \ S_{Hn-1}(T_{Hn-1})] \ \ldots (57)$$

For example, mixed gas includes 90 vol% of methane ($CH_4$), 5 vol% of ethane ($C_2H_6$), 1 vol% of propane ($C_3H_8$), 1 vol% of butane ($C_4H_{10}$), 1 vol% of nitrogen ($N_2$), and 2 vol% of carbon dioxide ($CO_2$). In this case, since the number of carbon atoms in methane is 1, the product of the number of carbon atoms in methane, the atomic weight of carbon atoms, and the volume fraction of methane is 10.80963 (=1x12.0107x0.9). Further, since the number of carbon atoms in ethane is 2, the product of the number of carbon atoms in ethane, the atomic weight of carbon atoms, and the volume fraction of ethane is 1.20107 (=2x12.0107x0.05). Further, since the number of carbon atoms in propane is 3, the product of the number of carbon atoms in propane, the atomic weight of carbon atoms, and the volume fraction of propane is 0.360321 (=3x12.0107x0.01). Further, since the number of carbon atoms in butane is 4, the product of the number of carbon atoms in butane, the atomic weight of carbon atoms, and the volume fraction of butane is 0.480428 (=4x12.0107x0.01). The number of carbon atoms in nitrogen is 0. Since the number of carbon atoms in carbon dioxide is 1, the product of the number of carbon atoms in carbon dioxide, the atomic weight of carbon atoms, and the volume fraction of carbon dioxide is 0.240214 (=1x12.0107x0.02).

As a result, the sum of: the product of the number of carbon atoms in methane, the atomic weight of carbon atoms, and the volume fraction of methane; the product of the number of carbon atoms in ethane, the atomic weight of carbon atoms, and the volume fraction of ethane; the product of the number of carbon atoms in propane, the atomic weight of carbon atoms, and the volume fraction of propane; the product of the number of carbon atoms in butane, the atomic weight of carbon atoms, and the volume fraction of butane; and the product of the number of carbon atoms in carbon dioxide, the atomic weight of carbon atoms, and the volume fraction of carbon dioxide is 13.091663 (=10.80963+1.20107+0.360321+0.980428+0.240214).

Note that, as shown in Equations (48) to (51), ethane ($C_2H_6$), butane ($C_4H_{10}$), pentane ($C_5H_{12}$), or hexane ($C_6H_{14}$) may be considered as a mixture of methane ($CH_4$) and propane ($C_3H_8$), the mixture being multiplied by a predetermined coefficient, and Equation (57) may be calculated.

In the second embodiment, the equation creating section 302 of Fig. 15 collects: the known value of the sum of the product of the number $N_C$ of carbon atoms in each kind of molecules included in the first sample mixed gas, the atomic weight of carbon atoms, and the volume fraction of each kind of molecules in the mixed gas; the known value of the sum of the product of the number $N_C$ of carbon atoms in each kind of molecules included in the second sample mixed gas, the atomic weight of carbon atoms, and the volume fraction of each kind of molecules in the mixed gas; the known value of the sum of the product of the number $N_C$ of carbon atoms in each kind of molecules included in the third sample mixed gas, the atomic weight of carbon atoms, and the volume fraction of each kind of molecules in the mixed gas; the known value of the sum of the product of the number $N_C$ of carbon atoms in each kind of molecules included in the fourth sample mixed gas, the atomic weight of carbon atoms, and the volume fraction of each kind of molecules in the mixed gas; and the plurality of measured values of the electric signals $S_{H1}(T_{H1})$, $S_{H2}(T_{H2})$, $S_{H3}(T_{H3})$ output from the heating element 61, for example. Further, the equation creating section 302 calculates an equation based on the collected values of the sum of the product and based on the collected values of the electric signals $S_H$ by means of multivariate statistics. The equation created by the equation creating section 302 includes the electric signals $S_{H1}(T_{H1})$, $S_{H2}(T_{H2})$, $S_{H3}(T_{H3})$ output from the heating element 61, as independent variables. The equation further includes the sum of the product of the number $N_C$ of carbon atoms in each kind of molecules included in the gas, the atomic weight of carbon atoms, and the volume fraction of each kind of molecules, as a dependent variable. The equation creating section 302 stores the created equation in the equation storage device 402.

Further, in the second embodiment, the product calculating section 305 substitutes the measured values of the electric signals $S_{H1}(T_{H1})$, $S_{H2}(T_{H2})$, $S_{H3}(T_{H3})$ output from the heating element 61 in the independent variables in the equation stored in the equation storage device 402. The independent variables indicate the electric signals $S_{H1}(T_{H1})$, $S_{H2}(T_{H2})$, $S_{H3}(T_{H3})$ output from the heating element 61. The product calculating section 305 calculates the sum of the product of the number $N_C$ of carbon atoms in each kind of molecules included in the measuring-target mixed gas, the atomic weight of carbon atoms, and the volume fraction of each kind of molecules. The product calculating section 305 stores the sum of the product in the product storage device 403.

As described above, according to the second embodiment of the present invention, it is possible to measure the sum of the product of the number $N_C$ of carbon atoms in each kind of molecules included in the measuring-target mixed gas, the atomic weight of carbon atoms, and the volume fraction of each kind of molecules, based on the values of the electric signals $S_{H1}(T_{H1})$, $S_{H2}(T_{H2})$, $S_{H3}(T_{H3})$ output from the heating element 61 exposed to the measuring-target mixed gas.

(Example of second embodiment)

Similar to the first example of the first embodiment, 40 kinds of sample mixed gas were prepared. Compositions of the 40 kinds of prepared sample mixed gas were known. Next, each of the 40 kinds of sample mixed gas was used. A plurality of measured values of an electric signal $S_I$ from the first temperature detector 62 of Fig. 1, and a plurality of measured values of the electric signals $S_{H1}(T_{H1})$, $S_{H2}(T_{H2})$, $S_{H3}(T_{H3})$, $S_{H4}(T_{H4})$, $S_{H5}(T_{H5})$ output from the heating element 61 were obtained.

After that, an equation was created by means of support vector regression based on the known value of the sum of the product of the number of carbon atoms in each kind of molecules included in each of the 40 kinds of sample mixed gas, the atomic weight of carbon atoms, and the volume fraction of each kind of molecules, based on the plurality of measured values of the electric signal $S_I$ output from the first temperature detector 62, and based on the plurality of measured values of the electric signals $S_{H1}(T_{H1})$, $S_{H2}(T_{H2})$, $S_{H3}(T_{H3})$, $S_{H4}(T_{H4})$, $S_{H5}(T_{H5})$ output from the heating element 61. The electric signal $S_I$ output from the first temperature detector 62, and the electric signals $S_{H1}(T_{H1})$, $S_{H2}(T_{H2})$, $S_{H3}(T_{H3})$, $S_{H4}(T_{H4})$, $S_{H5}(T_{H5})$ output from the heating element 61 were the independent variables. The sum of the product of the number of carbon atoms in each kind of molecules included in the gas, the atomic weight of carbon atoms, and the volume fraction of each kind of molecules was a dependent variable.

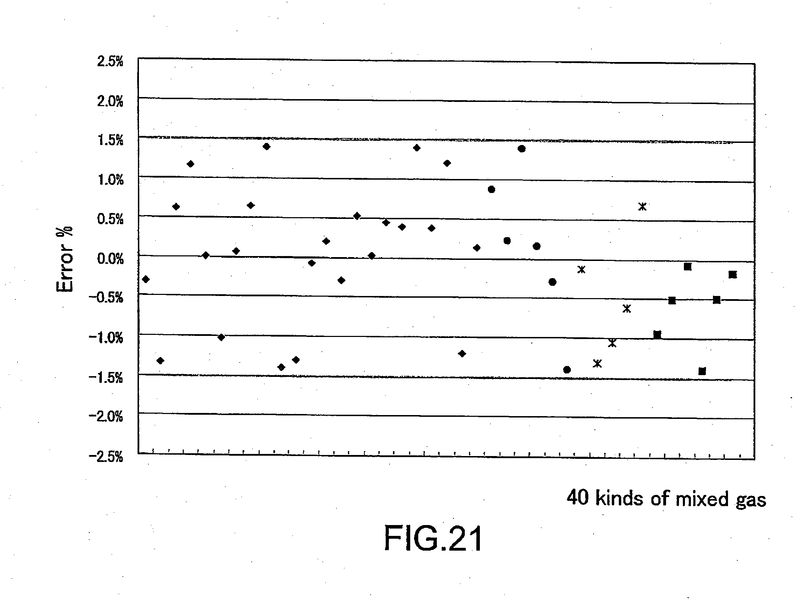

In creating the equation, the number of calibration points may be arbitrarily determined (e.g., 3 to 5). The sum of the product of the number of carbon atoms in each kind of molecules included in each of the 40 kinds of sample mixed gas, the atomic weight of carbon atoms, and the volume fraction of each kind of molecules was calculated by using the created equation. The sum was compared with the true value. As shown in Fig. 21, an error was plus/minus 1.5%.

(Third Embodiment)

The atmosphere gas around the microchip 8 of Fig. 1 to Fig. 4 is mixed gas including four kinds of gas components, i.e., the gas A, the gas B, the gas C, and the gas D. $K_{ACH}$ is indicative of a calorific value per unit volume of heat produced by carbon atoms and hydrogen atoms in the gas A. $K_{BCH}$ is indicative of a calorific value per unit volume of heat produced by carbon atoms and hydrogen atoms in the gas B. $K_{CCH}$ is indicative of a calorific value per unit volume of heat produced by carbon atoms and hydrogen atoms in the gas C. $K_{DCH}$ is indicative of a calorific value per unit volume of heat produced by carbon atoms and hydrogen atoms in the gas D. In this case, a calorific value $Q_{CH}$ per unit volume of heat produced by carbon atoms and hydrogen atoms in the mixed gas equals to the sum of values, which are obtained by multiplying the volume fractions of the gas components by the calorific values of heat produced by carbon atoms and hydrogen atoms in the gas components, per unit volume. In view of this, the following Equation (52) shows the calorific value $Q_{CH}$ per unit volume of heat produced by carbon atoms and hydrogen atoms in the mixed gas.

$$Q_{CH}=K_{ACH} \times V_A + K_{BCH} \times V_B + K_{CCH} \times V_C + K_{DCH} \times V_D \quad \cdots (58)$$

In view of Equation (58) and Equations (19) to (22), the following Equation (59) shows the calorific value $Q_{CH}$ per unit volume of heat produced by carbon atoms and hydrogen atoms in molecules included in the mixed gas. $g_4$ is indicative of a function.

$$Q_{CH} = g_4 [M_{I1}(T_{H1}), \ M_{I2}(T_{H2}), \ M_{I3}(T_{H3})] \ \ldots (59)$$

Further, as described above, the heat-radiation coefficient $M_I$ of the mixed gas depends on the electric signals $S_{H1}(T_{H1})$, $S_{H2}(T_{H2})$, $S_{H3}(T_{H3})$ output from the heating element 61 in the case where the heating temperatures of the heating element 61 are $T_{H1}$, $T_{H2}$, and $T_{H3}$, respectively, and depends on the electric signal $S_I$ output from the first temperature detector 62 exposed to the mixed gas. In view of this, the following Equation (60) also shows the calorific value $Q_{CH}$ per unit volume of heat produced by carbon atoms and hydrogen atoms in molecules included in the mixed gas. $h_{22}$ is indicative of a function.

$$Q_{CH} = h_{22} [S_{H1}(T_{H1}), \ S_{H2}(T_{H2}), \ S_{H3}(T_{H3}), \ S_I] \ \ldots (60)$$

Further, as shown in the following Equation (61), the independent variable of the electric signal $S_I$ output from the first temperature detector 62 may be omitted. $h_{23}$ is indicative of a function.

$$Q_{CH} = h_{23} [S_{H1}(T_{H1}), \ S_{H2}(T_{H2}), \ S_{H3}(T_{H3})] \ \ldots (61)$$

Further, the calorific value $Q_{CH}$ per unit volume of heat produced by carbon atoms and hydrogen atoms in molecules included in the mixed gas is in correlation with the sum of: the sum of the product of the number $N_C$ of carbon atoms in each kind of molecules included in the mixed gas, the atomic weight of carbon atoms, and the volume fraction of each kind of molecules; and the sum of the product of the number $N_H$ of hydrogen atoms in each kind of molecules included in the mixed gas, the atomic weight of hydrogen atoms, and the volume fraction of each kind of molecules. In view of this, the following Equation (62) shows the sum $G_{CH}$ of: the sum of the product of the number $N_C$ of carbon atoms in each kind of molecules included in the mixed gas, the atomic weight of carbon atoms, and the volume fraction of each kind of molecules; and the sum of the product of the number $N_H$ of hydrogen atoms in each kind of molecules included in the mixed gas, the atomic weight of hydrogen atoms, and the volume fraction of each kind of molecules, based on Equation (61). $h_{24}$ is indicative of a function.

$$G_{CH} = h_{24} [S_{H1}(T_{H1}), \ S_{H2}(T_{H2}), \ S_{H3}(T_{H3})] \ \ldots (62)$$

Further, the following Equation (63) shows the sum $G_{CH}$ of: the sum of the product of the number $N_C$ of carbon atoms in each kind of molecules included in the mixed gas, which includes n kinds of gas components, the atomic weight of carbon atoms, and the volume fraction of each kind of molecules; and the sum of the product of the number $N_H$ of hydrogen atoms in each kind of molecules included in the mixed gas, the atomic weight of hydrogen atoms, and the volume fraction of each kind of molecules. $h_{25}$ is indicative of a function.

$$G_{CH} = h_{25} [S_{H1}(T_{H1}), \ S_{H2}(T_{H2}), \ S_{H3}(T_{H3}), \ \ldots, \ S_{Hn-1}(T_{Hn-1})] \ \ldots (63)$$

Note that, as shown in Equations (48) to (51), ethane ($C_2H_6$), butane ($C_4H_{10}$), pentane ($C_5H_{12}$), or hexane ($C_6H_{14}$) may be considered as a mixture of methane ($CH_4$) and propane ($C_3H_8$), the mixture being multiplied by a predetermined coefficient, and Equation (63) may be calculated.

In a third embodiment, the equation creating section 302 of Fig. 15 collects: the known value of the sum of the product of the number $N_C$ of carbon atoms in each kind of molecules included in the first sample mixed gas, the atomic weight of carbon atoms, and the volume fraction of each kind of molecules; and the known value of the sum of the product of the number $N_H$ of hydrogen atoms in each kind of molecules included in the first sample mixed gas, the atomic weight of hydrogen atoms, and the volume fraction of each kind of molecules. Further, the equation creating section 302 of Fig. 15 collects: the known value of the sum of the product of the number $N_C$ of carbon atoms in each kind of molecules included in the second sample mixed gas, the atomic weight of carbon atoms, and the volume fraction of each kind of molecules; and the known value of the sum of the product of the number $N_H$ of hydrogen atoms in each kind of molecules included in the second sample mixed gas, the atomic weight of hydrogen atoms, and the volume fraction of each kind of molecules.

Further, the equation creating section 302 collects: the known value of the sum of the product of the number $N_C$ of carbon atoms in each kind of molecules included in the third sample mixed gas, the atomic weight of carbon atoms, and the volume fraction of each kind of molecules; and the known value of the sum of the product of the number $N_H$ of hydrogen atoms in each kind of molecules included in the third sample mixed gas, the atomic weight of hydrogen atoms, and the volume fraction of each kind of molecules. Further, the equation creating section 302 collects: the known value of the sum of the product of the number $N_C$ of carbon atoms in each kind of molecules included in the fourth sample mixed gas, the atomic weight of carbon atoms, and the volume fraction of each kind of molecules; and the known value of the sum of the product of the number $N_H$ of hydrogen atoms in each kind of molecules included in the fourth sample mixed gas, the atomic weight of hydrogen atoms, and the volume fraction of each kind of molecules.

Further, the equation creating section 302 collects the plurality of measured values of the electric signals $S_{H1}(T_{H1})$, $S_{H2}(T_{H2})$, $S_{H3}(T_{H3})$ output from the heating element 61. The equation creating section 302 calculates an equation based on the collected values of the sum of the product and based on the collected values of the electric signals $S_H$ by means of multivariate statistics. The equation created by the equation creating section 302 includes the electric signals $S_{H1}(T_{H1})$, $S_{H2}(T_{H2})$, $S_{H3}(T_{H3})$ output from the heating element 61, as independent variables. The equation created by the equation creating section 302 further includes the sum of: the sum of the product of the number $N_C$ of carbon atoms in each kind of molecules included in the gas, the atomic weight of carbon atoms, and the volume fraction of each kind of molecules; and the sum of the product of the number $N_H$ of hydrogen atoms in each kind of molecules included in the gas, the atomic weight of hydrogen atoms, and the volume fraction of each kind of molecules, as a dependent variable. The equation creating section 302 stores the created equation in the equation storage device 402.

Further, in the third embodiment, the product calculating section 305 substitutes the measured values of the electric signals $S_{H1}(T_{H1})$, $S_{H2}(T_{H2})$, $S_{H3}(T_{H3})$ output from the heating element 61 in the independent variables in the equation stored in the equation storage device 402. The independent variables indicate the electric signals $S_{H1}(T_{H1})$, $S_{H2}(T_{H2})$, $S_{H3}(T_{H3})$ output from the heating element 61. The product calculating section 305 calculates the sum of: the sum of the product of the number $N_C$ of carbon atoms in each kind of molecules included in the measuring-target mixed gas, the atomic weight of carbon atoms, and the volume fraction of each kind of molecules; and the sum of the product of the number $N_H$ of hydrogen atoms in each kind of molecules included in the measuring-target mixed gas, the atomic weight of hydrogen atoms, and the volume fraction of each kind of molecules. The product calculating section 305 stores the calculated sum in the product storage device 403.

As described above, according to the third embodiment of the present invention, it is possible to measure the sum of: the sum of the product of the number $N_C$ of carbon atoms in each kind of molecules included in the measuring-target mixed gas, the atomic weight of carbon atoms, and the volume fraction of each kind of molecules; and the sum of the product of the number $N_H$ of hydrogen atoms in each kind of molecules included in the measuring-target mixed gas, the atomic weight of hydrogen atoms, and the volume fraction of each kind of molecules, based on the values of the electric signals $S_{H1}(T_{H1})$, $S_{H2}(T_{H2})$, $S_{H3}(T_{H3})$ output from the heating element 61 exposed to the measuring-target mixed gas.

(Fourth Embodiment)

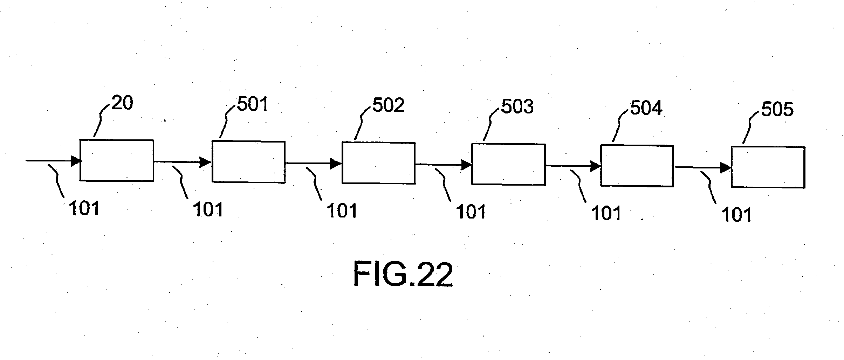

As shown in Fig. 22, an electric power generating system according to a fourth embodiment includes the gas measuring system 20, a flow controller 501, a reformer 502, a shifter 503, a selective oxidizer 504, and a fuel cell 505, which are connected in series in this order via the pipe 101. Gas is supplied to the gas measuring system 20. As described in the first embodiment, the gas measuring system 20 calculates the sum of the product of the number of hydrogen atoms in each kind of molecules included in the gas flowing in the pipe 101, the atomic weight of hydrogen atoms, and the volume fraction of each kind of molecules.

The flow controller 501 is arranged in the downstream of the gas measuring system 20. The flow controller 501 controls the flow rate of the gas flowing in the pipe 101 based on the sum of the product calculated by the gas measuring system 20. For example, the sum of the product calculated by the gas measuring system 20 is large. In this case, there are a lot of hydrogen molecules to be supplied to the fuel cell 505. So the flow rate of the gas flowing in the pipe 101 may be decreased. In contrast, the sum of the product calculated by the gas measuring system 20 is small. In this case, hydrogen molecules to be supplied to the fuel cell 505 may be insufficient. So the flow rate of the gas flowing in the pipe 101 may be increased.

The reformer 502 is arranged in the downstream of the flow controller 501. The reformer 502 adds water to the gas flowing in the pipe 101, and generates hydrogen molecules by means of reforming process, specifically, steam reforming. For example, methane in the gas reacts with water. As a result, carbon monoxide, carbon dioxide, and hydrogen are generated. The shifter 503 is arranged in the downstream of the reformer 502. The shifter 503 causes carbon monoxide in the gas to react with water. The shifter 503 generates carbon dioxide and hydrogen molecules by means of shift reaction to thereby reduce the concentration of carbon monoxide in the gas.

The selective oxidizer 504 is arranged in the downstream of the shifter 503. The selective oxidizer 504 causes carbon monoxide to react with oxygen, which remain in the gas to thereby generate carbon dioxide. As a result, the concentration of carbon monoxide in the gas is further reduced. The fuel cell 505 is arranged in the downstream of the selective oxidizer 504. Gas is supplied to the fuel cell 505. The gas supplied to the fuel cell 505 includes a lot of hydrogen molecules, and carbon monoxide the concentration of which is reduced. The fuel cell 505 generates electric power.

According to the electric power generating system of the fourth embodiment described above, it is possible to predict the molecular weight of hydrogen molecules supplied to the fuel cell 505. Further, it is possible to keep the molecular weight of hydrogen molecules supplied to the fuel cell 505 constant. As a result, it is possible to drive the fuel cell 505 stably. Note that the gas measuring system 20 may further calculate the sum of the product of the number of carbon atoms in each kind of molecules included in the gas, the atomic weight of carbon atoms, and the volume fraction of each kind of molecules.

**Claims**

1. A gas measuring system (20), comprising:

   a heating element (61) configured to produce heat at a plurality of heating temperatures ($T_H$), the heating element being arranged for exposure to gas;
   a measuring section (301) configured to measure values of electric signals ($S_H$) output from the heating element (61) at the plurality of heating temperatures ($T_H$), respectively;
   an equation storage device (402) in which an equation is stored, the equation including independent variables and a dependent variable, the independent variables being electric signals ($S_H$) output from the heating element (61) at the plurality of heating temperatures ($T_H$), respectively, the dependent variable being a sum of the product of the number of atoms in each kind of molecules included in the gas, atomic weight of the atoms, and a volume fraction of the each kind of molecules; and
   a calculating section (305) configured to substitute values of electric signals ($S_H$) output from the heating element (61) in the independent variables ($S_H$) of the equation, respectively, to calculate the sum of the product of the number of atoms in each kind of molecules included in the gas, atomic weight of the atoms, and a volume fraction of the each kind of molecules.

2. The gas measuring system (20) according to claim 1, wherein
   the sum of the product of the number of atoms in each kind of molecules included in the gas, atomic weight of the atoms, and a volume fraction of the each kind of molecules is a sum of the product of the number ($N_H$) of hydrogen atoms in each kind of molecules included in the gas, atomic weight of the hydrogen atoms, and a volume fraction of the each kind of molecules.

3. The gas measuring system (20) according to claim 1, wherein
   the sum of the product of the number of atoms in each kind of molecules included in the gas, atomic weight of the atoms, and a volume fraction of the each kind of molecules is a sum of the product of the number ($N_C$) of carbon atoms in each kind of molecules included in the gas, atomic weight of the carbon atoms, and a volume fraction of the each kind of molecules.

4. The gas measuring system (20) according to claim 1, wherein
   the sum of the product of the number of atoms in each kind of molecules included in the gas, atomic weight of the atoms, and a volume fraction of the each kind of molecules is a sum of
   a sum of the product of the number ($N_H$) of hydrogen atoms in each kind of molecules included in the gas, atomic weight of the hydrogen atoms, and a volume fraction of the each kind of molecules, and
   a sum of the product of the number ($N_C$) of carbon atoms in each kind of molecules included in the gas, atomic weight of the carbon atoms, and a volume fraction of the each kind of molecules.

5. An electric power generating system, comprising the gas measuring system (20) of claim 2;
   a fuel cell (505) configured to be supplied with hydrogen extracted from the gas; and
   a controller (501) configured to control a supply amount of the hydrogen supplied to the fuel cell (505) based on the calculated sum of the product.

**6.** The electric power generating system according to claim 5, wherein
the equation storage device (402) is configured to further store a second equation, the second equation including independent variables and a dependent variable, the independent variables being electric signals ($S_H$) output from the heating element (61) at the plurality of heating temperatures ($T_H$), respectively, the dependent variable being a sum of the product of the number ($N_C$) of carbon atoms in each kind of molecules included in the gas, atomic weight of the carbon atoms, and a volume fraction of the each kind of molecules, and
the calculating section (305) is configured to substitute values of electric signals ($S_H$) output from the heating element (61) in the independent variables of the second equation, respectively, to calculate the sum of the product of the number ($N_C$) of carbon atoms in each kind of molecules included in the gas, atomic weight of the carbon atoms, and a volume fraction of the each kind of molecules.

**7.** A gas measuring method, comprising the steps of:

exposing a heating element (61) to gas;
causing the heating element (61) to produce heat at a plurality of heating temperatures ($T_H$);
measuring values of electric signals ($S_H$) output from the heating element (61) at the plurality of heating temperatures ($T_H$), respectively;
providing an equation, the equation including independent variables and a dependent variable, the independent variables being electric signals ($S_H$) output from the heating element (61) at the plurality of heating temperatures ($T_H$), respectively, the dependent variable being a sum of the product of the number of atoms in each kind of molecules included in the gas, atomic weight of the atoms, and a volume fraction of the each kind of molecules; and
substituting values of electric signals ($S_H$) output from the heating element (61) in the independent variables of the equation, respectively, to calculate the sum of the product of the number of atoms in each kind of molecules included in the gas, atomic weight of the atoms, and a volume fraction of the each kind of molecules.

**8.** The gas measuring method according to claim 7, wherein
the sum of the product of the number of atoms in each kind of molecules included in the gas, atomic weight of the atoms, and a volume fraction of the each kind of molecules is a sum of the product of the number ($N_H$) of hydrogen atoms in each kind of molecules included in the gas, atomic weight of the hydrogen atoms, and a volume fraction of the each kind of molecules.

**9.** The gas measuring method according to claim 7, wherein
the sum of the product of the number of atoms in each kind of molecules included in the gas, atomic weight of the atoms, and a volume fraction of the each kind of molecules is a sum of the product of the number ($N_C$) of carbon atoms in each kind of molecules included in the gas, atomic weight of the carbon atoms, and a volume fraction of the each kind of molecules.

**10.** The gas measuring method according to claim 7, wherein
the sum of the product of the number of atoms in each kind of molecules included in the gas, atomic weight of the atoms, and a volume fraction of the each kind of molecules is a sum of
a sum of the product of the number ($N_H$) of hydrogen atoms in each kind of molecules included in the gas, atomic weight of the hydrogen atoms, and a volume fraction of the each kind of molecules, and
a sum of the product of the number ($N_C$) of carbon atoms in each kind of molecules included in the gas, atomic weight of the carbon atoms, and a volume fraction of the each kind of molecules.

**11.** A method of controlling an electric power generating system, comprising the steps of the gas measuring method of claim 8 and the further step of:

controlling a supply amount of hydrogen supplied to a fuel cell (505) based on the calculated sum of the product, the hydrogen being extracted from the gas.

**12.** The method of controlling an electric power generating system according to claim 11, further comprising:

providing a second equation, the second equation including independent variables and a dependent variable, the independent variables being electric signals ($S_H$) output from the heating element (61) at the plurality of heating temperatures ($T_H$), respectively, the dependent variable being a sum of the product of the number ($N_C$) of carbon atoms in each kind of molecules included in the gas, atomic weight of the carbon atoms, and a volume fraction of the each kind of molecules; and

substituting values of electric signals (SH) output from the heating element (61) in the independent variables of the second equation, respectively, to calculate the sum of the product of the number (Nc) of carbon atoms in each kind of molecules included in the gas, atomic weight of the carbon atoms, and a volume fraction of the each kind of molecules.

**Patentansprüche**

1. Gasmesssystem (20), umfassend:

ein Heizelement (61), welches eingerichtet ist, Wärme an einer Mehrzahl von Heiztemperaturen ($T_H$) zu erzeugen, wobei das Heizelement derart angeordnet ist, dass es Gas ausgesetzt ist;

ein Messabschnitt (301), der eingerichtet ist, Werte von elektrischen Signalen ($S_H$) zu messen, welche von dem Heizelement (61) jeweils an der Mehrzahl von Heiztemperaturen ($T_H$) ausgegeben werden;

eine Gleichungsspeichereinrichtung (402), in welcher eine Gleichung gespeichert ist, wobei die Gleichung unabhängige Variablen und eine abhängige Variable umfasst, wobei die unabhängigen Variablen die elektrischen Signale sind, die von dem Heizelement jeweils an der Mehrzahl der Heiztemperaturen ausgegeben werden, und wobei die abhängige Variable eine Summe ist der Produkte der Anzahl von Atomen in jeder Art von Molekülen, die in dem Gas enthalten sind, eines Atomgewichts der Atome und eines Volumenanteils jeder der Arten der Moleküle; und

einen Berechnungsabschnitt (305), der eingerichtet ist, Werte von elektrischen Signalen, die von dem Heizelement (61) ausgegeben werden, jeweils in die unabhängigen Variablen ($S_H$) der Gleichung zu substituieren, um die Summe der Produkte der Anzahl der Atome in jeder Art der Moleküle, die in dem Gas enthalten sind, eines Atomgewichts der Atome und eines Volumenanteils jeder der Arten der Moleküle zu berechnen.

2. Gasmesssystem (20) nach Anspruch 1, wobei die Summe der Produkte der Anzahl der Atome in jeder Art von Molekülen, die in den Gas enthalten sind, eines Atomgewichts der Atome und eines Volumenanteils jeder der Arten der Moleküle eine Summe ist der Produkte der Anzahl ($N_H$) von Wasserstoffatomen in jeder Art von Molekülen, die in dem Gas enthalten sind, eines Atomgewichts der Wasserstoffatome und eines Volumenanteils jeder der Arten der Moleküle.

3. Gasmesssystem (20) nach Anspruch 1, wobei die Summe der Produkte der Anzahl der Atome in jeder Art von Molekülen, die in den Gas enthalten sind, eines Atomgewichts der Atome und eines Volumenanteils jeder der Arten der Moleküle eine Summe ist der Produkte der Anzahl ($N_C$) von Kohlenstoffatomen in jeder Art von Molekülen, die in dem Gas enthalten sind, eines Atomgewichts der Kohlenstoffatome und eines Volumenanteils jeder der Arten der Moleküle.

4. Gasmesssystem (20) nach Anspruch 1, wobei die Summe der Produkte der Anzahl der Atome in jeder Art von Molekülen, die in den Gas enthalten sind, eines Atomgewichts der Atome und eines Volumenanteils jeder der Arten der Moleküle eine Summe ist

einer Summe der Produkte der Anzahl ($N_H$) von Wasserstoffatomen in jeder Art von Molekülen, die in dem Gas enthalten sind, eines Atomgewichts der Wasserstoffatome und eines Volumenanteils jeder der Arten der Moleküle, und

einer Summe der Produkte der Anzahl ($N_C$) von Kohlenstoffatomen in jeder Art von Molekülen, die in dem Gas enthalten sind, eines Atomgewichts der Kohlenstoffatome und eines Volumenanteils jeder der Arten der Moleküle.

5. Stromerzeugungssystem, umfassend das Gasmesssystem (20) nach Anspruch 2;

eine Brennstoffzelle (505), die eingerichtet ist, mit Wasserstoff versorgt zu werden, welcher aus dem Gas extrahiert wird; und

eine Steuereinheit (501), welche eingerichtet ist, eine Versorgungsmenge von Wasserstoff zu steuern, welche der Brennstoffzelle (505) zugeführt wird, basierend auf der berechneten Summe der Produkte.

6. Stromerzeugungssystem nach Anspruch 5, wobei

die Gleichungsspeichereinrichtung (402) eingerichtet ist, weiterhin eine zweite Gleichung zu speichern, wobei die zweite Gleichung unabhängige Variablen und eine abhängige Variable umfasst, wobei die unabhängigen Variablen elektrische Signale ($S_H$) sind, welche von dem Heizelement (61) jeweils an der Mehrzahl von Heiztemperaturen ($T_H$) ausgegeben werden, und wobei die abhängige Variable eine Summe ist der Produkte der Anzahl ($N_C$) von Kohlenstoffatomen in jeder Art von Molekülen, die in den Gas enthalten sind, eines Atomgewichts der Kohlenstoffatome und eines Volumenanteils jeder der Arten der Moleküle, und

der Berechnungsabschnitt (305) eingerichtet ist, Werte von elektrischen Signalen ($S_H$), welche von dem Heizelement (61) ausgegeben werden, jeweils in die unabhängigen Variablen der zweiten Gleichung zu substituieren, um die Summe der Produkte der Anzahl ($N_C$) von Kohlenstoffatomen in jeder Art von Molekülen, die in den Gas enthalten sind, eines Atomgewichts der Kohlenstoffatome und eines Volumenanteils jeder der Arten der Moleküle zu berechnen.

7. Gasmessverfahren, umfassend den Schritt:

   ein Heizelement (61) einem Gas aussetzen;
   das Heizelement veranlassen, Wärme an einer Mehrzahl von Heiztemperaturen ($T_H$) zu erzeugen;
   Messen von Werten von elektrischen Signalen ($S_H$), welche von dem Heizelement (61) an der Mehrzahl von Heiztemperaturen ($T_H$) jeweils erzeugt werden;
   Bereitstellen einer Gleichung, wobei die Gleichung unabhängige Variablen und
   eine abhängige Variable umfasst, wobei die unabhängigen Variablen elektrische Signale ($S_H$) sind, welche von dem Heizelement (61) an der Mehrzahl von Heiztemperaturen ($T_H$) jeweils ausgegeben werden, und wobei die abhängige Variable eine Summe ist der Produkte der Anzahl von Atomen in jeder Art von Molekülen, die in dem Gas enthalten sind, eines Atomgewichts der Atome und eines Volumenanteils jeder der Arten der Moleküle; und
   Substituieren von Werten elektrischer Signale ($S_H$), welche von dem Heizelement (61) ausgegeben werden, jeweils in die unabhängigen Variablen der Gleichung, um die Summe der Produkte der Anzahl von Atomen in jeder Art der Moleküle, die in den Gas enthalten sind, eines Atomgewichts der Atome und eines Volumenanteils jeder der Arten der Moleküle zu berechnen.

8. Gasmessverfahren nach Anspruch 7, wobei
   die Summe der Produkte der Anzahl der Atome in jeder Art von Molekülen, die in den Gas enthalten sind, eines Atomgewichts der Atome und eines Volumenanteils jeder der Arten der Moleküle eine Summe ist der Produkte der Anzahl ($N_H$) von Wasserstoffatomen in jeder Art von Molekülen, die in dem Gas enthalten sind, eines Atomgewichts der Wasserstoffatome und eines Volumenanteils jeder der Arten der Moleküle.

9. Gasmessverfahren nach Anspruch 7, wobei
   die Summe der Produkte der Anzahl der Atome in jeder Art von Molekülen, die in den Gas enthalten sind, eines Atomgewichts der Atome und eines Volumenanteils jeder der Arten der Moleküle eine Summe ist der Produkte der Anzahl ($N_c$) von Kohlenstoffatomen in jeder Art von Molekülen, die in dem Gas enthalten sind, eines Atomgewichts der Kohlenstoffatome und eines Volumenanteils jeder der Arten der Moleküle.

10. Gasmessverfahren nach Anspruch 7, wobei
    die Summe der Produkte der Anzahl der Atome in jeder Art von Molekülen, die in den Gas enthalten sind, eines Atomgewichts der Atome und eines Volumenanteils jeder der Arten der Moleküle eine Summe ist
    einer Summe der Produkte der Anzahl ($N_H$) von Wasserstoffatomen in jeder Art von Molekülen, die in dem Gas enthalten sind, eines Atomgewichts der Wasserstoffatome und eines Volumenanteils jeder der Arten der Moleküle, und
    einer Summe der Produkte der Anzahl ($N_c$) von Kohlenstoffatomen in jeder Art von Molekülen, die in dem Gas enthalten sind, eines Atomgewichts der Kohlenstoffatome und eines Volumenanteils jeder der Arten der Moleküle.

11. Verfahren zum Steuern eines Stromerzeugungssystems, umfassend den Schritt des Gasmessverfahrens nach Anspruch 8 und den weiteren Schritt des
    Steuerns einer Versorgungsmenge an Wasserstoff, welche einer Brennstoffzelle (505) zugeführt wird, basierend auf der berechneten Summe der Produkte, wobei der Wasserstoff aus dem Gas extrahiert wird.

12. Verfahren zum Steuern eines Stromerzeugungssystems nach Anspruch 11, weiter umfassend:

    Bereitstellen einer zweiten Gleichung, wobei die zweite Gleichung unabhängige Variablen und eine abhängige Variable umfasst, wobei die unabhängigen Variablen elektrische Signale ($S_H$) sind, welche von dem Heizelement (61) jeweils an der Mehrzahl der Heiztemperaturen ($T_H$) ausgegeben werden, und wobei die abhängige Variable eine Summe ist der Produkte der Anzahl ($N_c$) von Kohlenstoffatomen in jeder Art der Moleküle, die in dem Gas enthalten sind, eines Atomgewichts der Kohlenstoffatome und eines Volumenanteils jeder der Arten der Moleküle; und

Substituieren von Werten von elektrischen Signalen ($S_H$), die von dem Heizelement (61) ausgegeben werden, jeweils in die unabhängigen Variablen der zweiten Gleichung, um die Summe der Produkte der Anzahl ($N_c$) der Kohlenstoffatome in jeder Art der Moleküle, die in dem Gas enthalten sind, eines Atomgewichts der Kohlenstoffatome und eines Volumenanteils jeder der Arten der Moleküle zu berechnen.

**Revendications**

1. Système de mesure de gaz (20), comprenant :

un élément chauffant (61) configuré pour produire de la chaleur à une pluralité de températures de chauffage ($T_H$), l'élément chauffant étant agencé pour une exposition à du gaz ;
une partie de mesure (301) configurée pour mesurer des valeurs de signaux électriques ($S_H$) produits à partir de l'élément chauffant (61) respectivement à la pluralité de températures de chauffage ($T_H$) ;
un dispositif de stockage d'équation (402), dans lequel est stockée une équation, l'équation incluant des variables indépendantes et une variable dépendante, les variables indépendantes étant des signaux électriques ($S_H$) produits à partir de l'élément chauffant (61) respectivement à la pluralité de températures de chauffage ($T_H$), la variable dépendante étant une somme du produit du nombre d'atomes dans chaque type de molécules incluses dans le gaz, du poids atomique des atomes et d'une fraction volumique de chaque type de molécules, et
une partie de calcul (305) configurée pour substituer des valeurs de signaux électriques ($S_H$) produits à partir de l'élément chauffant (61) respectivement dans les variables indépendantes ($S_H$) de l'équation, afin de calculer la somme du produit du nombre d'atomes dans chaque type de molécules incluses dans le gaz, du poids atomique des atomes, et d'une fraction volumique du chaque type de molécules.

2. Système de mesure de gaz (20) selon la revendication 1, dans lequel
la somme du produit du nombre d'atomes dans chaque type de molécules incluses dans le gaz, du poids atomique des atomes, et d'une fraction volumique de chaque type de molécules est une somme du produit du nombre ($N_H$) d'atomes d'hydrogène dans chaque type de molécules incluses dans le gaz, du poids atomique des atomes d'hydrogène, et d'une fraction volumique du chaque type de molécules.

3. Système de mesure de gaz (20) selon la revendication 1, dans lequel
la somme du produit du nombre d'atomes dans chaque type de molécules incluses dans le gaz, du poids atomique des atomes, et d'une fraction volumique de chaque type de molécules est une somme du produit du nombre ($N_C$) d'atomes de carbone dans chaque type de molécules incluses dans le gaz, du poids atomique des atomes de carbone, et d'une fraction volumique du chaque type de molécules.

4. Système de mesure de gaz (20) selon la revendication 1, dans lequel
la somme du produit du nombre d'atomes dans chaque type de molécules incluses dans le gaz, du poids atomique des atomes, et d'une fraction volumique du chaque type de molécules est une somme de
une somme du produit du nombre ($N_H$) d'atomes d'hydrogène dans chaque type de molécules incluses dans le gaz, du poids atomique des atomes d'hydrogène, et d'une fraction volumique du chaque type de molécules, et
une somme du produit du nombre ($N_C$) d'atomes de carbone dans chaque type de molécules incluses dans le gaz, du poids atomique des atomes de carbone, et d'une fraction volumique du chaque type de molécules.

5. Système de génération d'énergie électrique, comprenant le système de mesure de gaz (20) selon la revendication 2, une pile à combustible (505) configurée pour être alimentée en hydrogène extrait du gaz, et
un contrôleur (501) configuré pour commander une quantité d'alimentation de l'hydrogène alimentée vers la pile à combustible (505) sur la base de la somme calculée du produit.

6. Système de génération d'énergie électrique selon la revendication 5, dans lequel
le dispositif de stockage d'équation (402) est configuré pour stocker en outre une seconde équation, la seconde équation incluant des variables indépendantes et une variable dépendante, les variables indépendantes étant des signaux électriques ($S_H$) produits à partir de l'élément chauffant (61) respectivement à la pluralité de températures de chauffage ($T_H$), la variable dépendante étant une somme du produit du nombre ($N_C$) d'atomes de carbone dans chaque type de molécules incluses dans le gaz, du poids atomique des atomes de carbone, et d'une fraction volumique du chaque type de molécules, et
la partie de calcul (305) est configurée pour substituer des valeurs de signaux électriques ($S_H$) produits à partir de l'élément chauffant (61) respectivement dans les variables indépendantes ($S_H$) de la seconde équation, afin de calculer la somme du produit du nombre ($N_C$) d'atomes de carbone dans chaque type de molécules incluses dans le gaz, du poids atomique des atomes de carbone, et d'une fraction volumique du chaque type de molécules.

7. Procédé de mesure de gaz, comprenant les étapes pour :

exposer un élément chauffant (61) à du gaz ;
amener l'élément chauffant (61) à produire de la chaleur à une pluralité de températures de chauffage ($T_H$) ;
mesurer des valeurs de signaux électriques ($S_H$) produits à partir de l'élément chauffant (61) respectivement à la pluralité de températures de chauffage ($T_H$) ;
fournir une équation, l'équation incluant des variables indépendantes et une variable dépendante, les variables indépendantes étant des signaux électriques ($S_H$) produits à partir de l'élément chauffant (61) respectivement à la pluralité de températures de chauffage ($T_H$), la variable dépendante étant une somme du produit du nombre d'atomes dans chaque type de molécules incluses dans le gaz, du poids atomique des atomes et d'une fraction volumique du chaque type de molécules, et
substituer des valeurs de signaux électriques ($S_H$) produits à partir de l'élément chauffant (61) respectivement dans les variables indépendantes de l'équation, afin de calculer la somme du produit du nombre d'atomes dans chaque type de molécules incluses dans le gaz, du poids atomique des atomes, et d'une fraction volumique du chaque type de molécules.

8. Procédé de mesure de gaz selon la revendication 7, dans lequel
la somme du produit du nombre d'atomes dans chaque type de molécules incluses dans le gaz, du poids atomique des atomes, et d'une fraction volumique du chaque type de molécules est une somme du produit du nombre ($N_H$) d'atomes d'hydrogène dans chaque type de molécules incluses dans le gaz, du poids atomique des atomes d'hydrogène, et d'une fraction volumique du chaque type de molécules.

9. Procédé de mesure de gaz selon la revendication 7, dans lequel
la somme du produit du nombre d'atomes dans chaque type de molécules incluses dans le gaz, du poids atomique des atomes, et d'une fraction volumique du chaque type de molécules est une somme du produit du nombre ($N_C$) d'atomes de carbone dans chaque type de molécules incluses dans le gaz, du poids atomique des atomes de carbone, et d'une fraction volumique du chaque type de molécules.

10. Procédé de mesure de gaz selon la revendication 7, dans lequel
la somme du produit du nombre d'atomes dans chaque type de molécules incluses dans le gaz, du poids atomique des atomes, et d'une fraction volumique de chaque type de molécules est une somme de
une somme du produit du nombre ($N_H$) d'atomes d'hydrogène dans chaque type de molécules incluses dans le gaz, du poids atomique des atomes d'hydrogène, et d'une fraction volumique du chaque type de molécules, et
une somme du produit du nombre ($N_C$) d'atomes de carbone dans chaque type de molécules incluses dans le gaz, du poids atomique des atomes de carbone, et d'une fraction volumique du chaque type de molécules.

11. Procédé de commande d'un système de génération d'énergie électrique, comprenant les étapes du procédé de mesure de gaz selon la revendication 8 ainsi que l'étape supplémentaire pour :

commander une quantité d'alimentation de l'hydrogène alimentée vers une pile à combustible (505) sur la base de la somme calculée du produit, l'hydrogène étant extrait du gaz.

12. Procédé de commande d'un système de génération d'énergie électrique selon la revendication 11, comprenant en outre l'étape pour :

fournir une seconde équation, la seconde équation incluant des variables indépendantes et une variable dépendante, les variables indépendantes étant des signaux électriques ($S_H$) produits à partir de l'élément chauffant (61) respectivement à la pluralité de températures de chauffage ($T_H$), la variable dépendante étant une somme du produit du nombre ($N_C$) d'atomes de carbone dans chaque type de molécules incluses dans le gaz, du poids atomique des atomes de carbone, et d'une fraction volumique du chaque type de molécules, et
substituer des valeurs de signaux électriques ($S_H$) produits à partir de l'élément chauffant (61) respectivement dans les variables indépendantes ($S_H$) de la seconde équation, afin de calculer la somme du produit du nombre ($N_C$) d'atomes de carbone dans chaque type de molécules incluses dans le gaz, du poids atomique des atomes de carbone, et d'une fraction volumique du chaque type de molécules.

FIG.1

8:Microchip

EP 2 645 098 B1

8:Microchip

FIG.2

FIG.3

8:Microchip

FIG.4

FIG.5

EP 2 645 098 B1

FIG.6

EP 2 645 098 B1

FIG.7

EP 2 645 098 B1

FIG.8

FIG.9

EP 2 645 098 B1

FIG.10

EP 2 645 098 B1

FIG.11

FIG.12

FIG.13

FIG.14

FIG.15

EP 2 645 098 B1

FIG.16

FIG.17

```
                    ┌──────────────┐
                    │    START     │
                    └──────┬───────┘
                           │
                           ▼
        ┌──────────────────────────────────────┐
        │   Prepare measuring-target mixed gas  │      S200
        └──────────────────────────────────────┘
                           │
    ┌──────────────────────▶
    │                      ▼
    │   ┌──────────────────────────────────────┐
    │   │         Output electric signal        │      S201
    │   └──────────────────────────────────────┘
    │                      │
    │                      ▼               S202
    │             ◇──────────────────────◇
    │  No    ◇                              ◇
    └───────◇   Is temperature changed?     ◇
             ◇                              ◇
                ◇──────────────────────◇
                           │
                           │ Yes
                           ▼
        ┌──────────────────────────────────────┐
        │             Retrieve equation         │      S203
        └──────────────────────────────────────┘
                           │
                           ▼
        ┌──────────────────────────────────────┐
        │             Calculate product         │      S204
        └──────────────────────────────────────┘
                           │
                           ▼
                    ┌──────────────┐
                    │     END      │
                    └──────────────┘
```

# FIG.18

FIG.19

FIG.20

40 kinds of mixed gas

Error %

40 kinds of mixed gas

FIG.21

EP 2 645 098 B1

EP 2 645 098 B1

FIG.22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H10284104 B
- JP 2002315224 A
- JP H05141999 B

### Non-patent literature cited in the description

- **A. J SMOLA ; B. SCHOLKOPF.** A Tutorial on Support Vector Regression. *NeuroCOLT Technical Report,* 1998